(19) Europäisches Patentamt
European Patent Office
Office européen des brevets

(11) EP 3 303 572 B1

(12) EUROPÄISCHE PATENTSCHRIFT

(45) Veröffentlichungstag und Bekanntmachung des Hinweises auf die Patenterteilung:
**07.08.2019 Patentblatt 2019/32**

(21) Anmeldenummer: **16726571.9**

(22) Anmeldetag: **02.06.2016**

(51) Int Cl.:
***C12N 9/02*** (2006.01) ***C11D 3/386*** (2006.01)

(86) Internationale Anmeldenummer:
**PCT/EP2016/062440**

(87) Internationale Veröffentlichungsnummer:
**WO 2016/193335 (08.12.2016 Gazette 2016/49)**

(54) **WASCHMITTEL MIT VERBESSERTER WASCHLEISTUNG, ENTHALTEND MINDESTENS EINE LACCASE**

DETERGENTS WITH IMPROVED DETERGENT POWER, CONTAINING AT LEAST ONE LACCASE

DÉTERGENT CONTENANT AU MOINS UNE LACCASE, DONT LA PERFORMANCE DE LAVAGE EST AMÉLIORÉE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.06.2015 DE 102015210370**
**05.06.2015 DE 102015210369**
**05.06.2015 DE 102015210368**
**05.06.2015 DE 102015210367**

(43) Veröffentlichungstag der Anmeldung:
**11.04.2018 Patentblatt 2018/15**

(73) Patentinhaber: **Henkel AG & Co. KGaA**
**40589 Düsseldorf (DE)**

(72) Erfinder:
- **MUSSMANN, Nina**
**47877 Willich (DE)**
- **O'CONNELL, Timothy**
**40547 Düsseldorf (DE)**
- **HERBST, Daniela**
**40237 Düsseldorf (DE)**
- **PRÜSER, Inken**
**40215 Düsseldorf (DE)**
- **BERGER, Ralf G.**
**30455 Hannover (DE)**
- **LINKE, Diana**
**31547 Rehburg (DE)**
- **BEHRENS, Christoph**
**30167 Hannover (DE)**

(56) Entgegenhaltungen:
**WO-A1-97/11217** **WO-A2-99/49020**
**US-A1- 2014 356 913**

- **GIARDINA P ET AL: "Protein and gene structure of a blue laccase from Pleurotus ostreatus", BIOCHEM. JOURNAL,, Bd. 341, 1. Januar 1999 (1999-01-01), Seiten 655-663, XP008133386, & DATABASE EMBL [Online] 31. März 1998 (1998-03-31), "Pleurotus ostreatus mRNA for laccase (poxa1b gene)", gefunden im EBI accession no. EM_STD:AJ005018 Database accession no. AJ005018**
- **DATABASE UniProt [Online] 3. September 2014 (2014-09-03), "SubName: Full=Laccase {ECO:0000313|EMBL:KDQ26322.1};", XP002760681, gefunden im EBI accession no. UNIPROT:A0A067NQH1 Database accession no. A0A067NQH1**
- **RODRIGUEZ-COUTO SUSANA: "Fungal laccase in the textile industry", FUNGAL BIOMOLECULES: SOURCES, APPLICATIONS AND RECENT DEVELOPMENTS,, 1. Januar 2015 (2015-01-01), Seiten 63-72, XP009191286,**
- **TIAN LIQIANG ET AL: "Laccase-mediated system pretreatment to enhance the effect of hydrogen peroxide bleaching of cotton fabric", INTERNATIONAL JOURNAL OF BIOLOGICAL MACROMOLECULES, Bd. 50, Nr. 3, April 2012 (2012-04), Seiten 782-787, XP002760682,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 303 572 B1

- MARQUES DE SOUZA C G ET AL: "Purification and characterization of the main laccase produced by the white-rot fungus Pleurotus pulmonarius on wheat bran solid state medium", JOURNAL OF BASIC MICROBIOLOGY, WILEY - V C H VERLAG GMBH & CO. KGAA, BERLIN, DE, Bd. 43, Nr. 4, 1. Januar 2003 (2003-01-01) , Seiten 278-286, XP002378262, ISSN: 0233-111X, DOI: 10.1002/JOBM.200390031

SEQUENCE LISTING

<110> Henkel AG & Co. KGaA.

<120> Waschmittel mit verbesserter Waschleistung, enthaltend mindestens eine Laccase

<130> PT033256PCT

<150> DE 102015210367.6, DE 102015210369.2, DE 102015210368.4, DE 102015210370.6
<151> 2015-06-05

<160> 8

<170> PatentIn version 3.5

<210> 1
<211> 533
<212> PRT
<213> Pleurotus pulmonarius

<400> 1

```
Met Ala Val Ala Phe Ile Ala Leu Val Ser Leu Ala Leu Ala Leu Val
1               5                   10                  15

Arg Val Glu Ala Ser Ile Gly Pro Arg Gly Thr Leu Asn Ile Ala Asn
            20                  25                  30

Lys Val Ile Ser Pro Asp Gly Phe Ser Arg Ser Thr Val Leu Ala Gly
        35                  40                  45

Gly Ser Tyr Pro Gly Pro Leu Ile Lys Gly Lys Thr Gly Asp Arg Phe
    50                  55                  60

His Ile Asn Val Val Asn Glu Leu Ala Asp Thr Ser Met Pro Ile Asp
65                  70                  75                  80

Thr Ser Ile His Trp His Gly Leu Phe Ala Lys Gly His Asn Trp Ala
                85                  90                  95

Asp Gly Pro Ala Met Val Thr Gln Cys Pro Ile Val Pro Gly His Ser
            100                 105                 110

Phe Leu Tyr Glu Phe Asp Val Pro Asp Gln Ala Gly Thr Phe Trp Tyr
        115                 120                 125

His Ser His Leu Gly Thr Gln Tyr Cys Asp Gly Leu Arg Gly Pro Leu
    130                 135                 140

Val Ile Tyr Ser Lys Asn Asp Pro His Lys Arg Leu Tyr Asp Val Asp
145                 150                 155                 160
```

```
Asp Glu Ser Thr Val Leu Thr Val Gly Asp Trp Tyr His Ala Pro Ser
                165                 170                 175

Leu Ser Leu Thr Gly Val Pro His Pro Asp Ser Thr Leu Phe Asn Gly
            180                 185                 190

Leu Gly Arg Ser Leu Asn Gly Pro Ala Ser Pro Leu Tyr Val Met Asn
        195                 200                 205

Val Val Lys Gly Lys Arg Tyr Arg Ile Arg Leu Ile Asn Thr Ser Cys
    210                 215                 220

Asp Ser Asn Tyr Gln Phe Ser Ile Asp Gly His Thr Phe Thr Val Ile
225                 230                 235                 240

Glu Ala Asp Gly Glu Asn Thr Gln Pro Leu Gln Val Asp Gln Val Gln
                245                 250                 255

Ile Phe Ala Gly Gln Arg Tyr Ser Leu Val Leu Asn Ala Asn Gln Ala
            260                 265                 270

Val Gly Asn Tyr Trp Ile Arg Ala Asn Pro Asn Ser Gly Asp Pro Gly
        275                 280                 285

Phe Ala Asn Gln Met Asn Ser Ala Ile Leu Arg Tyr Lys Gly Ala His
    290                 295                 300

Ser Ile Asp Pro Thr Thr Pro Glu Gln Asn Ala Thr Asn Pro Leu Arg
305                 310                 315                 320

Glu Tyr Asn Leu Arg Pro Leu Val Lys Lys Pro Ala Pro Gly Lys Pro
                325                 330                 335

Phe Pro Gly Gly Ala Asp His Asn Ile Asn Leu Asn Phe Ala Phe Asp
            340                 345                 350

Pro Ala Thr Ala Leu Phe Thr Ala Asn Asn Phe Thr Phe Val Pro Pro
        355                 360                 365

Thr Val Pro Val Leu Leu Gln Ile Leu Ser Gly Thr Arg Asp Ala His
    370                 375                 380

Asp Leu Ala Pro Ala Gly Ser Ile Tyr Asp Ile Lys Leu Gly Glu Val
385                 390                 395                 400

Val Glu Ile Thr Met Pro Ala Leu Val Phe Ala Gly Pro His Pro Leu
                405                 410                 415
```

```
His Leu His Gly His Thr Phe Ala Val Val Arg Ser Ala Gly Ser Ser
            420                 425                 430

Thr Tyr Asn Tyr Glu Asn Pro Val Arg Arg Asp Val Val Ser Ile Gly
        435                 440                 445

Asp Asp Pro Thr Asp Asn Val Thr Ile Arg Phe Val Ala Asp Asn Ala
    450                 455                 460

Gly Pro Trp Phe Leu His Cys His Ile Asp Trp His Leu Asp Leu Gly
465                 470                 475                 480

Phe Ala Val Val Phe Ala Glu Gly Val Asn Gln Thr Ala Val Ala Asn
                485                 490                 495

Pro Val Pro Glu Ala Trp Asn Asn Leu Cys Pro Leu Tyr Asn Ser Ser
            500                 505                 510

Asn Pro Ser Lys Leu Leu Met Gly Thr Asn Ala Ile Gly Arg Leu Pro
        515                 520                 525

Ala Pro Leu Lys Ala
    530
```

<210> 2
<211> 1602
<212> DNA
<213> Pleurotus pulmonarius

<400> 2

```
atggcggttg cattcattgc gcttgtttca cttgccttag cactcgtacg cgtcgaggcc      60
agcatcgggc cccgcggaac gctgaacata gcgaacaaag tcatcagccc agatggattt     120
tctcgctcaa ccgtgctcgc cggtggctcc tacccgggcc cgttgatcaa ggggaagacc     180
ggtgataggt ttcatatcaa tgtcgtgaac gagctcgccg atacgtcgat gccaatcgac     240
accagtatcc actggcatgg tctcttcgcc aagggccaca attgggcaga tggccctgcc     300
atggttacgc aatgcccaat cgttccgggc cactcgtttt tgtacgaatt cgacgtccct     360
gatcaagctg gaacattttg gtatcactcc catctgggga cacagtactg tgatggactg     420
cgagggccat tggtcatcta ctcgaagaac gacccccaca agcgtttgta cgatgtcgac     480
gatgaatcca cggtgctgac cgttggtgac tggtaccacg ccccctcatt atcacttact     540
ggagtgcccc atcccgactc aacactcttc aatggcctcg gacgttccct caacggtcca     600
gcatcgccgc tatacgttat gaacgtagtc aaaggcaaac gctatcgtat tcggctcatc     660
aacacctcct gcgactccaa ctaccaattt tccattgacg gacatacctt cactgttatc     720

gaagctgatg gggagaacac acagcctctg caggtcgatc aagtgcaaat tttgcaggc      780
cagcgctatt cgcttgtcct caacgcgaat caggcggtcg gcaactactg gatccgcgca     840
aaccccaaca gcggcgatcc cggtttcgcg aaccagatga actctgccat cctccggtac     900
aaaggcgcac acagcatcga ccccacgacc cccgagcaga acgctaccaa ccccctccgc     960
gaatacaacc tccgcccgct cgtcaagaag cccgcgccag gcaaaccatt ccccggaggc    1020
gccgaccaca acatcaacct aaacttcgct ttcgatcctg ccaccgctct gttcactgcg    1080
aacaatttca cgtttgtccc ccctactgtt ccagtattgt tgcagatctt gtcgggcaca    1140
cgcgatgcgc atgatttggc ccctgctggg tcgatttatg acattaagct aggagaagtc    1200
gttgagatca ctatgcctgc acttgtattt gctggaccac atccccttca tttacatggg    1260
catacettcg ctgtagttcg cagtgccggc agcagcacat ataactacga gaatcccgtc    1320
cgtagagatg ttgtatccat cggtgatgac ccaacggaca acgtcaccat ccgattcgta    1380
gcagacaacg caggtccttg gttcctgcac tgccacatcg actggcatct tgacctcggc    1440
ttcgcggtcg tctttgccga aggagtaaat cagaccgcag tagccaaccc cgtacctgaa    1500
gcctggaaca acctctgccc cctatacaac agctctaacc catctaaact tctaatgggc    1560
actaatgcca ttggccgtct gcctgcgcca ctgaaggcat ga                       1602
```

<210> 3
<211> 521
<212> PRT
<213> Pleurotus ostreatus var. florida

<400> 3

```
Met Val Leu Ser Thr Lys Leu Ala Ala Leu Val Ala Ser Leu Pro Phe
1               5                   10                  15

Val Leu Ala Ala Thr Lys Lys Leu Asp Phe His Ile Arg Asn Asp Val
            20                  25                  30

Val Ser Pro Asp Gly Phe Glu Arg Arg Ala Ile Thr Val Asn Gly Ile
        35                  40                  45

Phe Pro Gly Thr Pro Val Ile Leu Gln Lys Asn Asp Lys Val Gln Ile
    50                  55                  60

Asn Thr Ile Asn Glu Leu Thr Asp Pro Gly Met Arg Arg Ser Thr Ser
65                  70                  75                  80

Ile His Trp His Gly Leu Phe Gln His Lys Thr Ser Gly Met Asp Gly
                85                  90                  95
```

```
Pro Ser Phe Val Asn Gln Cys Pro Ile Pro Pro Asn Ser Thr Phe Leu
            100                 105                 110

Tyr Asp Phe Asp Thr Ala Gly Gln Thr Gly Asn Tyr Trp Tyr His Ser
        115                 120                 125

His Leu Ser Thr Gln Tyr Cys Asp Gly Leu Arg Gly Ser Phe Ile Val
    130                 135                 140

Tyr Asp Pro Asn Asp Pro Leu Lys His Leu Tyr Asp Val Asp Asp Glu
145                 150                 155                 160

Ser Thr Ile Ile Thr Leu Ala Asp Trp Tyr His Asp Leu Ala Pro His
                165                 170                 175

Ala Gln Asn Gln Phe Phe Gln Thr Gly Ser Val Pro Ile Pro Asp Thr
            180                 185                 190

Gly Leu Ile Asn Gly Val Gly Arg Phe Lys Gly Gly Pro Leu Val Pro
        195                 200                 205

Tyr Ala Val Ile Asn Val Glu Gln Gly Lys Arg Tyr Arg Phe Arg Leu
    210                 215                 220

Ile Gln Ile Ser Cys Arg Pro Phe Phe Thr Phe Ser Ile Asp Asn His
225                 230                 235                 240

Thr Phe Asp Ala Ile Glu Phe Asp Gly Ile Glu His Asp Pro Thr Pro
                245                 250                 255

Ala Gln Asn Ile Asp Ile Tyr Ala Ala Gln Arg Ala Ser Ile Ile Val
            260                 265                 270

His Ala Asn Gln Thr Ile Asp Asn Tyr Trp Ile Arg Ala Pro Leu Thr
        275                 280                 285

Gly Gly Asn Pro Ala Gly Asn Pro Asn Leu Asp Ile Ser Leu Ile Arg
    290                 295                 300

Ala Ile Leu Arg Tyr Lys Gly Ala Pro Ala Val Glu Pro Thr Thr Val
305                 310                 315                 320

Ala Thr Thr Gly Gly His Lys Leu Asn Asp Ala Glu Met His Pro Ile
                325                 330                 335

Ala Gln Glu Gly Pro Gly Asn Leu Gly Thr Gly Pro Pro Asp Met Ala
            340                 345                 350
```

```
Ile Thr Leu Asn Ile Ala Gln Pro Asn Pro Pro Phe Phe Asp Ile Asn
        355                 360                 365

Gly Ile Ser Tyr Leu Ser Pro Ser Val Pro Val Leu Leu Gln Met Leu
    370                 375                 380

Ser Gly Ala Arg Lys Pro Gln Asp Phe Leu Pro Ser Glu Gln Val Ile
385                 390                 395                 400

Ile Leu Pro Ala Asn Lys Leu Ile Glu Val Ser Ile Pro Gly Ala Gly
                405                 410                 415

Ala His Pro Phe His Leu His Gly His Thr Phe Asp Ile Val Arg Thr
            420                 425                 430

Ser Asn Ser Asp Val Val Asn Leu Val Asn Pro Pro Arg Arg Asp Val
        435                 440                 445

Leu Pro Ile Asn Gly Gly Asn Thr Thr Phe Arg Phe Phe Ser Gly Asn
    450                 455                 460

Ser Gly Ala Trp Phe Leu His Cys His Ile Asp Trp His Leu Glu Ala
465                 470                 475                 480

Gly Leu Ala Val Val Phe Ala Glu Arg Pro Ala Glu Val Asn Glu Gly
                485                 490                 495

Glu Gln Ala Gln Ile Val Thr Gln Asp Trp Arg Thr Leu Cys Pro Ala
            500                 505                 510

Tyr Asp Gly Leu Ala Pro Glu Phe Gln
        515                 520
```

<210> 4
<211> 1566
<212> DNA
<213> Pleurotus ostreatus var. florida

<400> 4

```
atggtgctct ctactaagct cgctgctctt gtggcttcgc tccccttcgt tcttgctgcg        60
acgaagaaac ttgacttcca cattcgaaac gacgttgtct caccagacgg cttcgagcgc       120
agggcgatca cagtcaatgg catcttccct gggacgcccg ttatactgca gaagaacgac       180
aaggtccaga tcaatactat caatgaactg acggaccctg gcatgcgtcg cagtacctcc       240
atccattggc acggtttatt ccaacataaa acctccggca tggacgggcc ttctttcgtt       300
aaccagtgcc cgattccccc caactctact ttcctttacg acttcgatac cgcaggacag       360
actggaaact actggtacca ctcccacttg tctacgcaat attgcgacgg tcttcgtggt       420
tctttcatcg tttatgatcc caatgatcct ctgaagcacc tgtacgatgt tgatgatgag       480
agcaccatca tcaccctggc agattggtat catgaccttg ctccccacgc tcaaaaccag       540
ttcttccaaa ctggttcggt cccgattccc gacactggcc tgatcaacgg tgttggacga       600
ttcaagggcg gtcctcttgt cccttacgcc gtcatcaacg ttgagcaggg caagcgatac       660
agattccgcc tcatccagat ttcatgtcgt cccttcttca ccttctctat tgacaaccac       720
acattcgatg caattgaatt cgacggcata gagcacgacc cgacacccgc ccagaacatt       780
gatatctatg ctgctcaacg tgcatccatc atcgtccacg ccaaccagac catcgacaac       840
tactggatcc gtgcgccact cacaggggga aaccccgcag gtaaccctaa cctggacata       900
tcgttgatca gggccatcct tcgttacaag ggtgctcccg ctgtcgagcc caccacagtt       960
gcgactactg gaggccacaa gctcaatgat gccgagatgc acccaattgc tcaggaaggt      1020
cccggaaact tgggcaccgg ccccccagac atggccatca ccttgaacat cgcccagccg      1080
aaccctccat tcttcgatat caacggcatt tcgtatctct ctccttcggt tccagtgttg      1140
ctccagatgc ttagcggtgc tcgcaaacca caagacttcc tgccatcgga acaagtcatc      1200
atcttgcccg ccaacaagct cattgaagtc tctatacccg gtgccggcgc gcatcctttc      1260
catttgcacg gtcatacctt cgacattgtc cgcacttcga atagcgacgt cgtgaacctt      1320
gtcaacccac cgcgtcgcga cgtattgcct atcaacggtg gaaacacaac attccgtttc      1380
ttctctggca actccggtgc atggttcctc cattgccata ttgactggca tcttgaagct      1440
ggtctcgctg ttgtcttcgc ggaacgacct gctgaagtga acgagggcga acaggctcag      1500
atcgtcaccc aagactggag gacgctttgc ccagcttatg atggcctcgc tcccgagttc      1560
cagtaa                                                                  1566
```

<210> 5
<211> 515
<212> PRT
<213> Lentinula edodes

<400> 5

```
Met Gly Phe Gln Ser Phe Thr Ser Leu Leu Ser Leu Val Ala Leu Val
1               5                   10                  15

Pro Ser Ala Phe Ala Val Leu Glu Thr Thr Gly Asp Leu Val Ile Ser
            20                  25                  30

Asn Ala Ala Val Ser Pro Asp Gly Phe Ser Arg Thr Ala Val Leu Ala
        35                  40                  45

Gly Gly Gly Val Val Gly Glu Leu Val Thr Gly Asn Lys Gly Asp Asn
```

```
        50                  55                  60

Phe Gln Ile Asn Val Val Asn Glu Leu Thr Asp Asp Thr Met Leu Arg
65                  70                  75                  80

Ser Thr Thr Val His Trp His Gly Ile Phe Gln Glu Gly Thr Asn Trp
                85                  90                  95

Ala Asp Gly Pro Ala Phe Ile Asn Gln Cys Pro Ile Ala Ala Asn Asn
            100                 105                 110

Ser Phe Leu Tyr Asp Phe Thr Val Ser Asp Gln Ala Gly Thr Phe Trp
        115                 120                 125

Tyr His Ser His Leu Ala Thr Gln Tyr Cys Asp Gly Leu Arg Gly Pro
    130                 135                 140

Met Val Ile Tyr Asp Pro Asp Asp Pro Tyr Ala Asp Leu Tyr Asp Val
145                 150                 155                 160

Asp Asp Asp Thr Thr Ile Ile Ser Leu Met Asp Trp Tyr His Ala Lys
                165                 170                 175

Ala Glu Thr Leu Thr Phe Pro Ala Pro Asp Ala Thr Leu Ile Asn Gly
            180                 185                 190

Leu Gly Arg Tyr Ser Gly Gly Asn Ala Thr Asp Leu Ala Val Ile Thr
        195                 200                 205

Val Thr Ser Gly Thr Arg Tyr Arg Phe Arg Leu Ile Asn Leu Ala Cys
    210                 215                 220

Asp Pro Asn Phe Val Phe Ser Ile Asp Asn His Thr Met Thr Val Ile
225                 230                 235                 240

Glu Val Asp Gly Val Asn His Glu Gln Leu Val Val Asp Ser Ile Gln
                245                 250                 255

Ile Phe Ala Ala Gln Arg Tyr Ser Phe Ile Leu Glu Ala Asn Gln Ser
            260                 265                 270

Val Asp Asn Tyr Trp Ile Arg Ala Asp Pro Ser Thr Gly Thr Ala Gly
        275                 280                 285

Tyr Thr Gly Gly Ile Asn Ser Ala Ile Leu Arg Tyr Ser Gly Ala Ala
    290                 295                 300
```

Asp Ser Glu Pro Gly Thr Pro Ala Val Thr Ser Val Leu Ala Leu Asn
305 310 315 320

Glu Thr Gln Leu Val Pro Leu Glu Asn Pro Gly Ala Pro Gly Glu Pro
325 330 335

Glu Val Gly Gly Val Asp Tyr Ala Leu Asn Leu Ala Met Ala Phe Thr
340 345 350

Gly Thr Ala Phe Thr Ile Asn Gly Tyr Ser Phe Thr Ser Pro Thr Val
355 360 365

Pro Val Leu Leu Gln Ile Leu Ser Gly Ala Glu Thr Ala Asp Ser Leu
370 375 380

Leu Pro Thr Gly Ser Val Tyr Ser Leu Pro Thr Asn Ser Thr Ile Glu
385 390 395 400

Ile Ser Ile Pro Ser Gly Gly Leu Ala Gly Phe Pro His Pro Phe His
405 410 415

Leu His Gly His Thr Phe Asp Val Val Arg Ser Ala Gly Ser Thr Thr
420 425 430

Tyr Asn Tyr Val Asn Pro Val Arg Arg Asp Val Val Ser Thr Gly Ala
435 440 445

Ala Gly Asp Asn Val Thr Ile Arg Phe Thr Thr Asp Asn Ala Gly Pro
450 455 460

Trp Phe Leu His Cys His Ile Asp Trp His Leu Glu Ala Gly Leu Ala
465 470 475 480

Ile Val Phe Ala Glu Asp Val Ala Asp Trp Asn Thr Thr Gln Thr Pro
485 490 495

Ser Thr Ala Trp Asp Asp Leu Cys Pro Ile Tyr Asp Ala Leu Ser Ala
500 505 510

Asp Gln Leu
515

<210> 6
<211> 1548
<212> DNA
<213> Lentinula edodes

<400> 6

```
atgggctttc aatcttttac ctccctcctt tcactggtcg cacttgttcc gtcagctttt      60

gctgtcctgg aaacaactgg tgacctcgtc atttccaacg ctgcagtcag ccccgatggt     120
ttcagccgta ccgctgtcct tgctggcggt ggggtcgtag gtgaacttgt cacaggaaac     180
aagggcgata acttccaaat caatgttgtg aacgagttga cggacgatac catgttgcga     240
tctacaaccg tccattggca cggcatcttc caagagggaa ccaattgggc tgatggacct     300
gcattcatca atcagtgtcc catagcagca aacaactcct tcttatacga cttcaccgtt     360
tctgatcaag cagggacatt ttggtatcac agccatctcg cgacgcaata ttgcgatggt     420
cttcgaggtc ctatggtcat ctatgatcct gatgatcctt atgcggactt gtacgatgtt     480
gacgacgaca ccaccattat cagtctcatg gattggtatc acgcaaaggc tgaaactctc     540
accttcccgg ctccggatgc taccttaatc aacggtcttg gacgttactc tggaggtaat     600
gccactgatt tggctgtcat cacagtgact tcagggactc gataccgatt ccgcttaatt     660
aatttggctt gtgatcccaa cttcgtgttc agcattgata atcatacgat gactgtcatc     720
gaagtagatg gagtcaacca cgagcaatta gtcgttgatt ctatccagat tttcgctgcc     780
caacgatact cattcattct tgaagctaat caatccgtgg acaattattg gattcgcgcg     840
gacccctcaa ccggtaccgc tggatataca ggagggatca attctgccat tctgcgatac     900
agtggagccg ccgacagtga gcctggaact ccggcagtta caagtgttct ggctctaaac     960
gaaactcagc ttgtgccttt ggaaaaccct ggtgctccag gagaacctga agtaggaggc    1020
gtagattatg ctttaaatct ggctatggca tttactggaa cggctttcac tatcaatggc    1080
tatagcttta cttcaccgac ggtacccgtc ttgcttcaaa tcctcagcgg tgcggaaacg    1140
gccgattcgc ttttgcctac ggggtctgtt tactccttgc ccaccaactc gaccatcgag    1200
atttctattc cttctggagg acttgccggt ttccctcacc ccttccatct tcacggtcat    1260
accttcgatg ttgtgcgcag tgctggtagt acgacctata actacgtcaa cccggtccgt    1320
cgagatgtcg tcagcactgg agccgccggg gacaatgtaa caatccgttt cactacagat    1380
aacgcgggtc cttggttcct tcactgccac attgactggc atttggaggc cggtcttgcc    1440
attgtctttg ctgaagatgt tgccgattgg aacactaccc agactccctc cactgcttgg    1500
gatgatctgt gccccatcta cgacgctctt tctgctgacc aactttga                  1548
```

<210> 7
<211> 520
<212> PRT
<213> Trametes species (Tsp, DSMZ 11309)

<400> 7

```
Met Ser Arg Phe Gln Ser Leu Leu Ala Phe Val Val Ala Ser Leu Ala
1               5                   10                  15
```

```
Ala Val Ala His Ala Ala Ile Gly Pro Thr Ala Asp Leu Thr Ile Ser
            20                  25                  30

Asn Ala Glu Val Ser Pro Asp Gly Phe Ala Arg Gln Ala Val Val Val
        35                  40                  45

Asn Asn Val Thr Pro Gly Pro Leu Val Ala Gly Asn Lys Gly Asp Arg
    50                  55                  60

Phe Gln Leu Asn Val Ile Asp Asn Leu Thr Asn His Thr Met Leu Lys
65                  70                  75                  80

Ser Thr Ser Ile His Trp His Gly Phe Phe Gln Lys Gly Thr Asn Trp
                85                  90                  95

Ala Asp Gly Pro Ala Phe Val Asn Gln Cys Pro Ile Ser Ser Gly His
            100                 105                 110

Ser Phe Leu Tyr Asp Phe Gln Val Pro Asp Gln Ala Gly Thr Phe Trp
        115                 120                 125

Tyr His Ser His Leu Ser Thr Gln Tyr Cys Asp Gly Leu Arg Gly Pro
    130                 135                 140

Phe Val Val Tyr Asp Pro Asn Asp Pro His Ala Ser Leu Tyr Asp Val
145                 150                 155                 160

Asp Asn Asp Asp Thr Val Ile Thr Leu Ala Asp Trp Tyr His Thr Ala
                165                 170                 175

Ala Lys Leu Gly Pro Ala Phe Pro Leu Gly Ala Asp Ala Thr Leu Ile
            180                 185                 190

Asn Gly Leu Gly Arg Ser Pro Ser Thr Thr Ala Ala Asp Leu Ala Val
        195                 200                 205

Ile Asn Val Thr Lys Gly Lys Arg Tyr Arg Phe Arg Leu Val Ser Leu
    210                 215                 220

Ser Cys Asp Pro Asn His Thr Phe Ser Ile Asp Gly His Asp Leu Thr
225                 230                 235                 240

Ile Ile Glu Val Asp Ser Ile Asn Ser Gln Pro Leu Val Val Asp Ser
                245                 250                 255

Ile Gln Ile Phe Ala Ala Gln Arg Tyr Ser Phe Val Leu Asn Ala Asp
            260                 265                 270
```

```
Gln Asp Val Gly Asn Tyr Trp Ile Arg Ala Asn Pro Asn Phe Gly Asn
        275                 280                 285

Val Gly Phe Ala Gly Gly Ile Asn Ser Ala Ile Leu Arg Tyr Asp Gly
    290                 295                 300

Ala Asp Pro Val Glu Pro Thr Thr Thr Gln Thr Thr Pro Thr Lys Pro
305                 310                 315                 320

Leu Asn Glu Val Asp Leu His Pro Leu Ala Thr Met Ala Val Pro Gly
                325                 330                 335

Ser Pro Val Ala Gly Gly Val Asp Thr Ala Ile Asn Met Ala Phe Asn
            340                 345                 350

Phe Asn Gly Thr Asn Phe Phe Ile Asn Gly Ala Ser Phe Val Pro Pro
        355                 360                 365

Thr Val Pro Val Leu Leu Gln Ile Ile Ser Gly Ala Gln Asn Ala Gln
    370                 375                 380

Asp Leu Leu Pro Ser Gly Ser Val Tyr Ser Leu Pro Ala Asn Ala Asp
385                 390                 395                 400

Ile Glu Ile Ser Phe Pro Ala Thr Ala Ala Ala Pro Gly Ala Pro His
                405                 410                 415

Pro Phe His Leu His Gly His Ala Phe Ala Val Val Arg Ser Ala Gly
            420                 425                 430

Ser Thr Val Tyr Asn Tyr Asp Asn Pro Ile Phe Arg Asp Val Val Ser
        435                 440                 445

Thr Gly Thr Pro Ala Ala Gly Asp Asn Val Thr Ile Arg Phe Arg Thr
    450                 455                 460

Asp Asn Pro Gly Pro Trp Phe Leu His Cys His Ile Asp Phe His Leu
465                 470                 475                 480

Glu Ala Gly Phe Ala Val Val Phe Ala Glu Asp Ile Pro Asp Val Ala
                485                 490                 495

Ser Ala Asn Pro Val Pro Gln Ala Trp Ser Asp Leu Cys Pro Ile Tyr
            500                 505                 510

Asp Ala Leu Asp Val Asn Asp Gln
        515                 520
```

<210> 8
<211> 1563
<212> DNA
<213> Trametes species (Tsp, DSMZ 11309)

<400> 8

```
atgtcgaggt ttcagtctct gctcgccttc gtcgtcgcct ccctcgcggc tgtggcccat      60
gccgccattg ggcccaccgc tgacctcacc atctccaatg ccgaggtcag ccccgatggg     120
ttcgctcgtc aggctgtggt tgtcaacaat gttaccccag gacccctcgt cgcgggcaac     180
aagggtgacc gcttccaact caatgtcatc gacaacctca cgaaccacac tatgctgaag     240
agcacgagta ttcactggca tggcttcttc cagaagggga caaactgggc tgatggtccc     300
gcgtttgtga accagtgccc tatttcctct gggcactcgt tcctctacga tttccaggtt     360
cccgaccagg ccggtacctt ctggtaccac agccacttgt ccactcagta ctgtgacggc     420
ctgcggggtc ctttcgttgt gtacgatccc aatgacccgc acgcgagctt gtatgacgtc     480
gacaatgacg acaccgtgat caccctcgcg gattggtacc acactgccgc gaagctcggc     540
cccgcgttcc cactgggcgc ggatgctacc cttatcaacg ggctcgggcg ctcccccagc     600
accacggcgg cggacctcgc ggtcatcaac gtcacgaagg gtaaacgcta ccgtttccgc     660
ctggtttccc tgtcgtgtga ccccaaccac acgttcagca tcgatggtca tgacttgacg     720
atcatcgagg tggactccat caattcccaa cctctggtgg ttgactccat ccagattttc     780
gctgcgcagc ggtactcctt tgtgttgaat gccgatcagg acgtcgggaa ctactggatt     840
cgcgccaacc ccaactttgg caacgtcgga tttgcggggg gtatcaactc ggccatcctg     900
cgctatgacg gcgccgaccc ggttgagccc accacgactc agactacgcc gaccaagccc     960
ctgaacgagg tcgacttgca cccgctcgcc accatggctg tgcccggctc cccggtcgca    1020
ggtggtgttg acacggctat caacatggcc ttcaacttta acggtaccaa ctttttcatc    1080
aacggcgcga gctttgtgcc ccccaccgtg ccggtcctgc tccagatcat cagcggcgcc    1140
caaaacgccc aggatctcct cccgtctggc agcgtctact ccctcccggc gaacgcggac    1200
atcgagatct cgttccctgc taccgcggct gctccgggtg cccctcaccc cttccacttg    1260
cacggtcacg ccttcgccgt cgttcgcagc gctggcagca ccgtctacaa ctacgacaac    1320
cccatcttcc gcgacgtcgt cagcacgggg acgcctgcgg ccggtgacaa cgtcaccatc    1380
cgcttccgca ccgacaaccc cggtccgtgg ttcctccact gccacatcga cttccacctc    1440
gaggccggct tcgcggtcgt gttcgcggag gacatccccg acgtcgcgtc ggcgaaccct    1500
gtccctcagg cgtggtctga tctgtgcccc atctacgacg cgctcgatgt caacgaccag    1560
tag                                                                  1563
```

## Beschreibung

Die vorliegende Erfindung betrifft die Verwendung bestimmter Laccasen beim Waschen von Textilien sowie Waschmittel, welche diese Laccasen enthalten.

Laccasen (EC 1.10.3.2) sind kupferhaltige, "blaue" Enzyme, die in vielen Pflanzen, Pilzen und Mikroorganismen vorkommen. Laccasen zählen zu den Oxidoreduktasen. Das katalytisch aktive Zentrum enthält vier Kupferionen, die nach ihren spektroskopischen Eigenschaften unterschieden werden können. Das "blaue" Typ 1-Kupfer ist an der Substratoxidation beteiligt, ein Typ 2- und zwei Typ 3-Kupferionen formen einen trinuklearen Cluster, der Sauerstoff bindet und zu Wasser reduziert. Laccasen werden auch als p-Diphenol-Oxidasen bezeichnet. Zusätzlich zu Diphenolen oxidieren Laccasen viele andere Substrate wie methoxysubstituierte Phenole und Diamine. In Bezug auf ihre Substrate sind Laccasen erstaunlich unspezifisch. Wegen ihrer breiten Substratspezifität und ihrer Fähigkeit, phenolische Verbindungen zu oxidieren, haben Laccasen großes Interesse bei industriellen Anwendungen geweckt. Viel versprechende Gebiete zur Anwendung von Laccasen schließen zum Beispiel die Delignifizierung und das Kleben von Faserplatten der Holzindustrie, das Färben von Stoffen und das Entgiften von Färbeabwässern in der Textilindustrie, sowie die Verwendung in Biosensoren ein.

Mit der Hilfe von Mediatoren, d. h. zwischengeschalteten Molekülen, können Laccasen auch Substrate oxidieren, die sie sonst nicht in der Lage wären zu oxidieren. Die Mediatoren sind typischerweise "Small Molecule Compounds", die durch Laccasen oxidiert werden. Der oxidierte Mediator oxidiert dann wiederum das tatsächliche Substrat.

Die erste Laccase wurde bereits 1883 im japanischen Lackbaum (Rhus vernicifera) gefunden. Laccasen finden sich in vielen Pflanzen wie Pfirsich, Tomate, Mango und Kartoffel; Laccasen sind auch aus einigen Insekten bekannt. Die am häufigsten eingesetzten Laccasen stammen jedoch aus Pilzen, beispielsweise aus den Arten Agaricus, Aspergillus, Cerrena, Curvularia, Fusarium, Lentinius, Monocillium, Myceliophtora, Neurospora, Penicillium, Phanerochaete, Phlebia, Pleurotus, Podospora, Schizophyllum, Sporotrichum, Stagonospora und Trametes.

In der Natur besteht die Funktion der Laccasen unter anderem in der Mitwirkung an der Dekompostierung von Lignozellulose, der Biosynthese von Zellwänden, dem Braunwerden von Früchten und Gemüsen, sowie der Vorbeugung gegen mikrobische Angriffe auf Pflanzen.

Es ist oft schwierig, farbige Flecken/Verschmutzungen effektiv aus verschmutzter Wäsche oder von einem verschmutzten Gegenstand zu entfernen. Stark farbige Flecken und Verschmutzungen, d. h. von Obst und/oder Gemüse stammende, sind beim Entfernen eine besondere Herausforderung. Diese Flecken und Verschmutzungen enthalten Farbkörper auf der Basis von Carotinoidverbindungen, wie a-, b- und g-Carotin und Lycopin, von Porphyrinen, wie Chlorophyll, und von Flavonoidpigmenten und Farbstoffbestandteilen. Diese letztere Gruppe von Farbstoffbestandteilen auf der Basis von natürlichem Flavonoid umfasst die stark farbigen Anthocyanin-Farbstoffe und - Pigmente auf der Basis von Pelargonidin, Cyanidin, Delphidin und deren Methylestern und die Antoxanthine. Diese Verbindungen sind der Ursprung der meisten orangefarbenen, roten, violetten und blauen Farben in Früchten und sind in allen Beeren, Kirsche, roten und schwarzen Johannisbeeren, Grapefruits, Maracuja, Orangen, Zitronen, Äpfeln, Birnen, Granatapfel, Rotkohl, roten Beten und auch in Blumen reichlich vorhanden. Derivate von Cyanidin sind in bis zu 80% der pigmentierten Blätter, in bis zu 70% der Früchte und in bis zu 50% der Blumen vorhanden. Zu speziellen Beispielen solcher Verschmutzungen gehören Verschmutzungen aus Tee, Kaffee, Gewürzen wie Curry und Paprika, Orange, Tomate, Banane, Tee, Mango, Brokkoli, Möhre, rote Bete, Spinat und Gras. Kugelschreibertinte ist auch als sehr schwer zu entfernende farbige Flecken bekannt. Diese stark farbigen Flavonoid- und Carotinoid-Farbstoffe sind oft polycyclische und heterocyclische Verbindungen mit Systemen einer konjugierten Doppelbindung. Diese chemische Struktur ist oft für die deutliche Farbe der Verbindungen verantwortlich.

Die genannten Schwierigkeiten bei der Entfernung farbiger Flecken und Verschmutzungen sind besonders gravierend bei der Formulierung flüssiger Wasch- und Reinigungsmittel, da diese üblicherweise keine Bleichmittel enthalten.

WO9711217 offenbart verschiedene Laccasen aus Pilzen zur Fleckbehandlung von Textilien.

WO9949020 beschreibt die Verwendung Phenoloxidierender Enzyme aus Stachybotrys zur Entfernung von Gemüseflecken auf Textilien.

Überraschenderweise wurde gefunden, daß eine bislang unbekannte Laccase SEQ. ID NO.1 aus dem Lungen-Seitling (Pleurotus pulmonarius), oder hierzu hinreichend ähnliche Laccasen (bezogen auf die Sequenzidentität), besonders für den Einsatz in flüssigen Wasch- oder Reinigungsmitteln geeignet und hinsichtlich der Waschleistung verbessert sind.

Ein erster Gegenstand der vorliegenden Erfindung ist daher eine Laccase, die eine Aminosäuresequenz umfasst, die zu der in SEQ ID NO. 1 angegebenen Aminosäuresequenz über deren Gesamtlänge zu mindestens 97,5% und zunehmend bevorzugt zu mindestens 98%, 98,5% und 99% identisch ist.

Ein weiterer Gegenstand der vorliegenden Erfindung ist eine Laccase, die durch eine Nukleinsäuresequenz kodiert wird, die zu der in SEQ ID NO. 2 angegebenen Nukleinsäuresequenz über deren Gesamtlänge zu mindestens 94,5% und zunehmend bevorzugt zu mindestens 95%, 95,5%, 96%, 96,5%, 97%, 97,5%, 98%, 98,5% und 99% identisch

ist.

Ein weiterer Gegenstand der vorliegenden Erfindung ist ein Waschmittel, das mindestens eine Laccase enthält, die eine Aminosäuresequenz umfasst, die zu der in SEQ ID NO. 1 angegebenen Aminosäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, oder eine Laccase, die durch eine Nukleinsäuresequenz kodiert wird, die zu der in SEQ ID NO. 2 angegebenen Nukleinsäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist.

In einer bevorzugten Ausführungsform umfasst das Wachmittel eine Laccase, die eine Aminosäuresequenz umfasst, die zu der in SEQ ID NO. 1 angegebenen Aminosäuresequenz über deren Gesamtlänge zu mindestens 97,5% und zunehmend bevorzugt zu mindestens 98%, 98,5% und 99% identisch ist, oder eine Laccase, die durch eine Nukleinsäuresequenz kodiert wird, die zu der in SEQ ID NO. 2 angegebenen Nukleinsäuresequenz über deren Gesamtlänge zu mindestens 94,5% und zunehmend bevorzugt zu mindestens 95%, 95,5%, 96%, 96,5%, 97%, 97,5%, 98%, 98,5% und 99% identisch ist.

Vorteilhafterweise weist das erfindungsgemäße Mittel eine verbesserte Waschleistung, insbesondere bei der Entfernung farbiger Flecken und Verschmutzungen auf. Besonders bevorzugt eignet sich das erfindungsgemäße Mittel zur Entfernung Anthocyan-haltiger Anschmutzungen.

SEQ ID NO. 1 ist die Sequenz einer Laccase aus dem Basidiomyceten Pleurotus pulmonarius (Lungen-Seitling, auch bekannt als Löffelförmiger Seitling oder Sommeraustern-Seitling); DSMZ 5331 (DSMZ: Deutsche Sammlung von Mikroorganismen und Zellkulturen).

SEQ ID NO. 2 ist die für die Laccase mit der SEQ ID NO. 1 kodierende cDNA, d.h. ohne Introns.

Weiterhin wurde überraschenderweise gefunden, dass auch die folgenden Laccasen besonders für den Einsatz in flüssigen Wasch- oder Reinigungsmitteln geeignet und hinsichtlich der Waschleistung verbessert sind:

a) eine Laccase [SEQ. ID NO.3] aus dem Basidiomyceten Pleurotus ostreatus var. florida oder hierzu hinreichend ähnliche Laccasen (bezogen auf die Sequenzidentität);
b) eine bislang unbekannte Laccase [SEQ. ID NO.5] aus dem Shiitake-Pilz (Lentinula edodes) oder hierzu hinreichend ähnliche Laccasen (bezogen auf die Sequenzidentität);
c) eine bislang unbekannte Laccase [SEQ. ID NO.7] aus Trametes species (Tsp, DSMZ 11309) (DSMZ: Deutsche Sammlung von Mikroorganismen und Zellkulturen), oder hierzu hinreichend ähnliche Laccasen (bezogen auf die Sequenzidentität).

Ein weiterer Gegenstand der vorliegenden Erfindung ist daher eine Laccase, die eine Aminosäuresequenz umfasst, die zu der in SEQ ID NO. 3, SEQ ID NO.5, und/oder SEQ ID NO.7 ange gebenen Aminosäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist.

Weitere Gegenstände der vorliegenden Erfindung sind daher Waschmittel, welche mindestens eine Laccase enthalten, die eine Aminosäuresequenz umfasst, die zu der in SEQ ID NO. 3, SEQ ID NO. 5 und/oder SEQ ID NO.7 angegebenen Aminosäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist.

Vorteilhafterweise weist das erfindungsgemäße Mittel eine verbesserte Waschleistung, insbesondere bei der Entfernung farbiger Flecken und Verschmutzungen auf. Besonders bevorzugt eignet sich das erfindungsgemäße Mittel zur Entfernung Anthocyan-haltiger Anschmutzungen.

SEQ ID NO. 3 ist die Sequenz einer Laccase aus Pleurotus ostreatus var. florida aus der Veröffentlichung Palmieri, G. et al. (2003) Atypical laccaseisoenzymes from coppersupplemented Pleurotus ostreatus cultures. EnzymeMicrob.Technol. 33(2-3), 220-230. Die Sequenz findet sich auf Seite 227 der Veröffentlichung.

SEQ ID NO. 5 ist die Sequenz einer Laccase aus dem Basidiomyceten Lentinula edodes (Shitake).

SEQ ID NO. 7 ist die Sequenz einer Laccase aus Trametes species (Tsp, DSMZ 11309) (DSMZ: Deutsche Sammlung von Mikroorganismen und Zellkulturen).

Ein weiterer Gegenstand der vorliegenden Erfindung ist ein Waschmittel, das mindestens eine Laccase enthält, die durch eine Nukleinsäuresequenz codiert wird, die zu der in SEQ ID NO. 4, SEQ ID NO. 6 oder SEQ ID NO. 8 angegebenen Nukleinsäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist.

SEQ ID NO. 4 ist die für die Laccase mit der SEQ ID NO. 3 kodierende cDNA, d.h. ohne Introns.

SEQ ID NO. 6 ist die für die Laccase mit der SEQ ID NO. 5 kodierende cDNA, d.h. ohne Introns.

SEQ ID NO. 8 ist die für die Laccase mit der SEQ ID NO. 7 kodierende cDNA, d.h. ohne Introns.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. In der vorliegenden Patentanmeldung wurden alle Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.
Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Die erfindungsgemäßen und die in den erfindungsgemäßen Waschmitteln einsetzbaren Laccasen sind aus Pflanzen, Pilzen und vorzugsweise aus Bakterien erhältlich, insbesondere aus Bacilli und Actinomyceten. Die die SEQ ID NO. 1 umfassende Laccase kann aus Pleurotus pulmonarius gewonnen werden. Die die SEQ ID NO.3 umfassende Laccase kann aus Pleurotus ostreatus var. florida gewonnen werden. Die die SEQ ID NO.5 umfassende Laccase kann aus Lentinula edodes gewonnen werden. Die die SEQ ID NO.7 umfassende Laccase kann aus Trametes species gewonnen werden.

Die natürlichen Produktionsmengen der Laccasen sind allerdings oft sehr niedrig. Es kann daher sinnvoll sein, die Produktion zu erhöhen, indem man Laccasegene in fremden Produktionswirten exprimiert.

Zu diesem Zweck verwendet man in der Regel Vektoren, die eine Nukleinsäure enthalten, die für eine erfindungsgemäße Laccase kodiert.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren kodieren können, so dass eine bestimmte Aminosäuresequenz von mehreren unterschiedlichen Nukleinsäuren kodiert werden kann. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz kodierende Nukleinsäuren problemlos ermitteln. Weiterhin können bei Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure kodierend führt das dazu, dass in dem Organismus ein Codon weniger effizient translatiert wird als ein synonymes

Codon, das für dieselbe Aminosäure kodiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine für eine erfindungsgemäße Laccase kodierende Nukleinsäure enthalten. Sie vermögen diese in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine für eine erfindungsgemäße Laccase kodierende Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer für eine erfindungsgemäße Laccase kodierende Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Bevorzugt wird eine für eine erfindungsgemäße Laccase kodierende Nukleinsäure oder ein eine solche Nukleinsäure enthaltender Vektor in einen Mikroorganismus transformiert, der dann als Wirtszelle dient. Alternativ können auch einzelne Komponenten, d.h. Nukleinsäure-Teile oder -Fragmente einer für eine erfindungsgemäße Laccase kodierenden Nukleinsäure derart in eine Wirtszelle eingebracht werden, dass die dann resultierende Wirtszelle eine solche Nukleinsäure oder einen solchen Vektor enthält. Dieses Vorgehen eignet sich besonders dann, wenn die Wirtszelle bereits einen oder mehrere Bestandteile einer solchen Nukleinsäure oder eines solchen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Laccasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Laccasen funktionell beeinflussen.

Für die Herstellung erfindungsgemäßer Laccasen besonders geeignet sind solche Wirtszellen, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise Escherichia coli wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Die genannten Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen anwendbar und führt dazu, dass sich durch den Einsatz solcher Mikroorganismen erfindungsgemäße Laccasen herstellen lassen.

Besonders bevorzugte Wirtszellen zur Gewinnung der erfindungsgemäßen Laccasen sind Bakterien, insbesondere solche, die ausgewählt sind unter den Gattungen Escherichia, Klebsiella, Bacillus, Staphylococcus, Corynebakterium, Arthrobacter, Streptomyces, Stenotrophomonas und Pseudomonas und insbesondere unter Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.

Die Wirtszelle kann aber auch eine eukaryontische Zelle sein, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen oder Lipasen, kann vorteilhaft sein. Ferner können sich beispielsweise thermophile pilzliche Expressionssysteme besonders zur Expression temperaturbeständiger Proteine oder Varianten eignen.

Die genannten Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise einer erfindungsgemäßen Laccase.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Laccase erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Laccase kann aus dem Fermentationsmedium geerntet werden. Ein solches Fermentationsverfahren ist gegenüber einer Isolation der Laccase aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder

mehreren geeigneten Sekretionsmarkern oder -mechanismen und/oder Transportsystemen, damit die Wirtszellen die Laccase in das Fermentationsmedium sezernieren. Ohne Sekretion kann alternativ die Isolation der Laccase aus der Wirtszelle, d.h. eine Aufreinigung derselben aus der Zellmasse, erfolgen, beispielsweise unter Einsatz konventioneller Verfahren der Enzymchemie wie Salzfällung, Ultrafiltration, Ionenaustauschchromatographie und hydrophober Interaktionschromatographie. Die Aufreinigung kann durch SDS-Polyacrylamidgelelektrophoresen überwacht werden. Die Enzymaktivität des gereinigten Enzyms bei verschiedenen Temperaturen und pH-Werten kann bestimmt werden; in ähnlicher Weise können das Molekulargewicht und der isoelektrische Punkt bestimmt werden.

Überraschenderweise wurde gefunden, daß die erfindungsgemäßen Laccasen, insbesondere die die SEQ ID NO. 1 umfassende Laccase und auch die die SEQ ID NO.3, SEQ ID NO.5 und SEQ ID NO.7 umfassende Laccase die Waschleistung insbesondere flüssiger Waschmittelformulierungen verbessern und nicht, wie von anderen Laccasen bekannt, die unerwünschte Verdunklung und damit Intensivierung von Flecken, insbesondere Tee- und Kaffeeflecken bewirken.

Die Konzentration der Laccasen im erfindungsgemäßen Waschmittel beträgt von 0,001 bis 0,15 Gew.-%, vorzugsweise von 0,005 bis 0,06 Gew.-%, bezogen auf aktives Protein.

Das erfindungsgemäße Waschmittel ist vorzugsweise im Temperaturbereich von 5°C bis 95°C, bevorzugt 20°C bis 60°C und besonders bevorzugt 30°C bis 40°C einsetzbar.

Das erfindungsgemäße Waschmittel kann zusätzliche Mediatoren enthalten, um die zu entfernenden Farbstoffe mit höherer Effizienz zu oxidieren. Erfindungsgemäß geeignete Mediatoren sind beispielsweise Tempo (2,2,6,6-Tetramethyl-1-Piperidinyloxy), HBT (1-Hydroxybenzotriazol), ABTS (2,2'-Azinobis-3-Ethylbenzthiazol-6-Sulphonat), NHA (N-Hydroxy-Acetanilid), 2,5-Xylidin, Ethanol, Kupfer, 4-Methylcatechol, N-Hydroxyphthalimid, Gallussäure, Gerbsäure, Quercetin, Syringasäure, Guaiacol, Dimethoxybenzyl alcohol, Phenol, Violursäure (Isonitrosobarbitursäure), Phenolrot, Bromphenolblau, Cellulose, p-Kumarinsäure, Rooibos, o-Kresol, Dichloroindophenol, Hydroxybenzotriazol, Cycloheximid oder Vanillin.

Ein weiterer Gegenstand der Erfindung ist die Verwendung einer erfindungsgemäßen Laccase zur Verbesserung der Waschleistung eines Waschmittels, insbesondere bei der Entfernung farbiger Flecken und Verschmutzungen, besonders bevorzugt bei der Entfernung Anthocyan-haltiger Anschmutzungen.

Ein weiterer Gegenstand der Erfindung ist ein Verfahren zum Waschen von Textilien in tensidhaltigen wäßrigen Lösungen, welches dadurch gekennzeichnet ist, dass man eine tensidhaltige wäßrige Lösung einsetzt, die mindestens eine erfindungsgemäße Laccase enthält.

Das Verfahren wird in seiner einfachsten Form dadurch realisiert, dass man reinigungsbedürftige Textilien mit der wäßrigen Flotte in Kontakt bringt, wobei man eine übliche Waschmaschine einsetzen oder die Wäsche mit der Hand durchführen kann. Es ist erfindungsgemäß bevorzugt, das Verfahren unter intensiver Belüftung der Waschflotte durchzuführen, wie dies bei Verwendung eines üblichen Haushaltsmaschinen-Waschprogramms der Fall ist.

Ein Waschmittel kann neben den genannten, zur Entfernung farbiger Flecken und Verschmutzungen geeigneten Laccasen, übliche mit diesem Bestandteil verträgliche Inhaltsstoffe enthalten. So kann es beispielsweise zusätzlich noch einen oder mehrere Farbübertragungsinhibitoren, diese dann vorzugsweise in Mengen von 0,1 Gew.-% bis 2 Gew.-%, insbesondere 0,2 Gew.-% bis 1 Gew.-%, enthalten, die in einer bevorzugten Ausgestaltung ausgewählt werden aus den Polymeren aus Vinylpyrrolidon, Vinylimidazol, Vinylpyridin-N-Oxid oder den Copolymeren aus diesen. Brauchbar sind sowohl Polyvinylpyrrolidone mit Molgewichten von 15 000 g/mol bis 50 000 g/mol wie auch Polyvinylpyrrolidone mit höheren Molgewichten von beispielsweise bis zu über 1 000 000 g/mol, insbesondere von 1 500 000 g/mol bis 4 000 000 g/mol, N-Vinylimidazol/N-Vinylpyrrolidon-Copolymere, Polyvinyloxazolidone, Copolymere auf Basis von Vinylmonomeren und Carbonsäureamiden, pyrrolidongruppenhaltige Polyester und Polyamide, gepfropfte Polyamidoamine und Polyethylenimine, Polyamin-N-Oxid-Polymere und Polyvinylalkohole. Eingesetzt werden können aber auch enzymatische Systeme, umfassend eine Peroxidase und Wasserstoffperoxid beziehungsweise eine in Wasser Wasserstoffperoxid-liefernde Substanz. Der Zusatz einer Mediatorverbindung für die Peroxidase, zum Beispiel eines Acetosyringons, eines Phenolderivats oder eines Phenotiazins oder Phenoxazins, ist in diesem Fall bevorzugt, wobei auch zusätzlich obengenannte polymere Farbübertragungsinhibitorwirkstoffe eingesetzt werden können. Polyvinylpyrrolidon weist vorzugsweise eine durchschnittliche Molmasse im Bereich von 10 000 g/mol bis 60 000 g/mol, insbesondere im Bereich von 25 000 g/mol bis 50 000 g/mol auf. Unter den Copolymeren sind solche aus Vinylpyrrolidon und Vinylimidazol im Molverhältnis 5:1 bis 1:1 mit einer durchschnittlichen Molmasse im Bereich von 5 000 g/mol bis 50 000 g/mol, insbesondere 10 000 g/mol bis 20 000 g/mol bevorzugt.

Waschmittel, die als insbesondere pulverförmige Feststoffe, in nachverdichteter Teilchenform, in granularer Form, als homogene Lösungen oder Suspensionen vorliegen können, können außer den erfindungsgemäß eingesetzten Laccasen im Prinzip alle bekannten und in derartigen Mitteln üblichen Inhaltsstoffe enthalten. Die erfindungsgemäßen Mittel können insbesondere Buildersubstanzen, oberflächenaktive Tenside, Bleichmittel auf Basis organischer und/oder anorganischer Persauerstoffverbindungen, Bleichaktivatoren, wassermischbare organische Lösungsmittel, Enzyme, Sequestrierungsmittel, Elektrolyte, pH-Regulatoren und weitere Hilfsstoffe, wie optische Aufheller, Vergrauungsinhibitoren, Schaumregulatoren sowie Farb- und Duftstoffe enthalten.

Die Mittel enthalten vorzugsweise ein Tensid oder mehrere Tenside, wobei insbesondere anionische Tenside, nichtionische Tenside und deren Gemische, aber auch kationische, zwitterionische und amphotere Tenside in Frage kommen.

Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkyl-aminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise $C_{12}$-$C_{14}$-Alkohole mit 3 EO oder 4 EO, $C_9$-$C_{11}$-Alkohole mit 7 EO, $C_{13}$-$C_{15}$-Alkohole mit 3 EO, 5 EO, 7 EO oder 8 EO, $C_{12}$-$C_{18}$-Alkohole mit 3 EO, 5 EO oder 7 EO und Mischungen aus diesen, wie Mischungen aus $C_{12}$-$C_{14}$-Alkohol mit 3 EO und $C_{12}$-$C_{18}$-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind (Talg-) Fettalkohole mit 14 EO, 16 EO, 20 EO, 25 EO, 30 EO oder 40 EO. Insbesondere in Mitteln für den Einsatz in maschinellen Verfahren werden üblicherweise extrem schaumarme Verbindungen eingesetzt. Hierzu zählen vorzugsweise $C_{12}$-$C_{18}$-Alkylpolyethylenglykol-polypropylenglykolether mit jeweils bei zu 8 Mol Ethylenoxid- und Propylenoxideinheiten im Molekül. Man kann aber auch andere bekannt schaumarme nichtionische Tenside verwenden, wie zum Beispiel $C_{12}$-$C_{18}$-Alkylpolyethyl-englykol-polybutylenglykolether mit jeweils bis zu 8 Mol Ethylenoxid- und Butylenoxideinheiten im Molekül sowie endgruppenverschlossene Alkylpolyalkylenglykolmischether. Besonders bevorzugt sind auch die hydroxylgruppenhaltigen alkoxylierten Alkohole, sogenannte Hydroxymischether. Zu den nichtionischen Tensiden zählen auch Alkylglykoside der allgemeinen Formel $RO(G)_x$ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl - die als analytisch zu bestimmende Größe auch gebrochene Werte annehmen kann - zwischen 1 und 10; vorzugsweise liegt x bei 1,2 bis 1,4. Ebenfalls geeignet sind Polyhydroxyfettsäureamide der Formel

$R^2$
|
$R^1$-CO-N-[Z] (III)

in der $R^1CO$ für einen aliphatischen Acylrest mit 6 bis 22 Kohlenstoffatomen, $R^2$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht.

Vorzugsweise leiten sich die Polyhydroxyfettsäureamide von reduzierenden Zuckern mit 5 oder 6 Kohlenstoffatomen, insbesondere von der Glucose ab. Zur Gruppe der Polyhydroxyfettsäureamide gehören auch Verbindungen der Formel

$R^4$-O-$R^5$
|
$R^3$-CO-N-[Z] (IV)

in der $R^3$ für einen linearen oder verzweigten Alkyl- oder Alkenylrest mit 7 bis 12 Kohlenstoffatomen, $R^4$ für einen linearen, verzweigten oder cyclischen Alkylenrest oder einen Arylenrest mit 2 bis 8 Kohlenstoffatomen und $R^5$ für einen linearen, verzweigten oder cyclischen Alkylrest oder einen Arylrest oder einen Oxy-Alkylrest mit 1 bis 8 Kohlenstoffatomen steht, wobei $C_1$-$C_4$-Alkyl- oder Phenylreste bevorzugt sind, und [Z] für einen linearen Polyhydroxyalkylrest, dessen Alkylkette mit mindestens zwei Hydroxylgruppen substituiert ist, oder alkoxylierte, vorzugsweise ethoxylierte oder propoxylierte Derivate dieses Restes steht. [Z] wird auch hier vorzugsweise durch reduktive Aminierung eines Zuckers wie Glucose,

Fructose, Maltose, Lactose, Galactose, Mannose oder Xylose erhalten. Die N-Alkoxy- oder N-Aryloxy-substituierten Verbindungen können durch Umsetzung mit Fettsäuremethylestern in Gegenwart eines Alkoxids als Katalysator in die gewünschten Polyhydroxyfettsäureamide überführt werden. Eine weitere Klasse bevorzugt eingesetzter nichtionischer Tenside, die entweder als alleiniges nichtionisches Tensid oder in Kombination mit anderen nichtionischen Tensiden, insbesondere zusammen mit alkoxylierten Fettalkoholen und/oder Alkylglykosiden, eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester. Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon. Als weitere Tenside kommen sogenannte Gemini-Tenside in Betracht. Hierunter werden im Allgemeinen solche Verbindungen verstanden, die zwei hydrophile Gruppen pro Molekül besitzen. Diese Gruppen sind in der Regel durch einen sogenannten "Spacer" voneinander getrennt. Dieser Spacer ist in der Regel eine Kohlenstoffkette, die lang genug sein sollte, dass die hydrophilen Gruppen einen ausreichenden Abstand haben, damit sie unabhängig voneinander agieren können. Derartige Tenside zeichnen sich im Allgemeinen durch eine ungewöhnlich geringe kritische Micellkonzentration und die Fähigkeit, die Oberflächenspannung des Wassers stark zu reduzieren, aus. In Ausnahmefällen werden unter dem Ausdruck Gemini-Tenside nicht nur derartig "dimere", sondern auch entsprechend "trimere" Tenside verstanden. Geeignete Gemini-Tenside sind beispielsweise sulfatierte Hydroxymischether oder Dimeralkohol-bis- und Trimeralkohol-tris-sulfate und -ethersulfate. Endgruppenverschlossene dimere und trimere Mischether zeichnen sich insbesondere durch ihre Bi- und Multifunktionalität aus. So besitzen die genannten endgruppenverschlossenen Tenside gute Netzeigenschaften und sind dabei schaumarm, so dass sie sich insbesondere für den Einsatz in maschinellen Wasch- oder Reinigungsverfahren eignen. Eingesetzt werden können aber auch Gemini-Polyhydroxyfettsäureamide oder Poly-Polyhydroxyfettsäureamide.

Geeignete anionische Tenside sind insbesondere Seifen und solche, die Sulfat- oder SulfonatGruppen enthalten. Als Tenside vom Sulfonat-Typ kommen vorzugsweise $C_9$-$C_{13}$-Alkylbenzolsulfonate, Olefinsulfonate, das heißt Gemische aus Alken- und Hydroxyalkansulfonaten sowie Disulfonaten, wie man sie beispielsweise aus $C_{12}$-$C_{18}$-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus $C_{12}$-$C_{18}$-Alkanen beispielsweise durch Sulfochlorierung oder Sulfoxidation mit anschließender Hydrolyse beziehungsweise Neutralisation gewonnen werden. Geeignet sind auch die Ester von $\alpha$-Sulfofettsäuren (Estersulfonate), zum Beispiel die $\alpha$-sulfonierten Methylester der hydrierten Kokos-, Palmkern- oder Talgfettsäuren, die durch $\alpha$-Sulfonierung der Methylester von Fettsäuren pflanzlichen und/oder tierischen Ursprungs mit 8 bis 20 C-Atomen im Fettsäuremolekül und nachfolgende Neutralisation zu wasserlöslichen Mono-Salzen hergestellt werden, in Betracht. Vorzugsweise handelt es sich hierbei um die $\alpha$-sulfonierten Ester der hydrierten Kokos-, Palm-, Palmkern- oder Talgfettsäuren, wobei auch Sulfonierungsprodukte von ungesättigten Fettsäuren, beispielsweise Ölsäure, in geringen Mengen, vorzugsweise in Mengen nicht oberhalb etwa 2 bis 3 Gew.-%, vorhanden sein können. Insbesondere sind $\alpha$-Sulfofettsäurealkylester bevorzugt, die eine Alkylkette mit nicht mehr als 4 C-Atomen in der Estergruppe aufweisen, beispielsweise Methylester, Ethylester, Propylester und Butylester. Mit besonderem Vorteil werden die Methylester der $\alpha$-Sulfofettsäuren (MES), aber auch deren verseifte Disalze eingesetzt. Weitere geeignete Aniontenside sind sulfierte Fettsäureglycerinester, welche Mono-, Di- und Triester sowie deren Gemische darstellen, wie sie bei der Herstellung durch Veresterung durch ein Monoglycerin mit 1 bis 3 Mol Fettsäure oder bei der Umesterung von Triglyceriden mit 0,3 bis 2 Mol Glycerin erhalten werden. Als Alk(en)ylsulfate werden die Alkali- und insbesondere die Natriumsalze der Schwefelsäurehalbester der $C_{12}$-$C_{18}$-Fettalkohole beispielsweise aus Kokosfettalkohol, Talgfettalkohol, Lauryl-, Myristyl-, Cetyl- oder Stearylalkohol oder der $C_{10}$-$C_{20}$-Oxoalkohole und diejenigen Halbester sekundärer Alkohole dieser Kettenlänge bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind $C_{12}$-$C_{16}$-Alkylsulfate und $C_{12}$-$C_{15}$-Alkylsulfate sowie $C_{14}$-$C_{15}$-Alkylsulfate insbesondere bevorzugt. Auch 2,3-Alkylsulfate, welche als Handelsprodukte der Shell Oil Company unter dem Namen DAN® erhalten werden können, sind geeignete Aniontenside. Geeignet sind auch die Schwefelsäuremonoester der mit 1 bis 6 Mol Ethylenoxid ethoxylierten geradkettigen oder verzweigten $C_7$-$C_{21}$-Alkohole, wie 2-Methylverzweigte $C_9$-$C_{11}$-Alkohole mit im Durchschnitt 3,5 Mol Ethylenoxid (EO) oder $C_{12}$-$C_{18}$-Fettalkohole mit 1 bis 4 EO. Zu den bevorzugten Aniontensiden gehören auch die Salze der Alkylsulfobernsteinsäure, die auch als Sulfosuccinate oder als Sulfobernsteinsäureester bezeichnet werden, und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten $C_8$-bis $C_{18}$-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder

deren Salze einzusetzen. Als weitere anionische Tenside kommen Fettsäure-Derivate von Aminosäuren, beispielsweise von N-Methyltaurin (Tauride) und/oder von N-Methylglycin (Sarkoside) in Betracht. Insbesondere bevorzugt sind dabei die Sarkoside beziehungsweise die Sarkosinate und hier vor allem Sarkosinate von höheren und gegebenenfalls einfach oder mehrfach ungesättigten Fettsäuren wie Oleylsarkosinat. Als weitere anionische Tenside kommen insbesondere Seifen in Betracht. Geeignet sind insbesondere gesättigte Fettsäureseifen, wie die Salze der Laurinsäure, Myristinsäure, Palmitinsäure, Stearinsäure, hydrierten Erucasäure und Behensäure sowie insbesondere aus natürlichen Fettsäuren, zum Beispiel Kokos-, Palmkern- oder Talgfettsäuren, abgeleitete Seifengemische. Zusammen mit diesen Seifen oder als Ersatzmittel für Seifen können auch die bekannten Alkenylbernsteinsäuresalze eingesetzt werden.

Die anionischen Tenside, einschließlich der Seifen, können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor. Tenside sind in Waschmitteln in Mengenanteilen von normalerweise 1 Gew.-% bis 50 Gew.-%, insbesondere von 5 Gew.-% bis 30 Gew.-%, enthalten.

Ein Waschmittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Glycindiessigsäure, Methylglycindiessigsäure, Nitrilotriessigsäure, Iminodisuccinate wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylenphosphonsäure), Lysintetra(methylenphosphonsäure) und 1-Hydroxyethan-1,1-diphosphonsäure, polymere Hydroxyverbindungen wie Dextrin sowie polymere (Poly-)carbonsäuren, insbesondere durch Oxidation von Polysacchariden zugängliche Polycarboxylate, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative mittlere Molekülmasse der Homopolymeren ungesättigter Carbonsäuren liegt im allgemeinen zwischen 5 000 g/mol und 200 000 g/mol, die der Copolymeren zwischen 2 000 g/mol und 200 000 g/mol, vorzugsweise 50 000 g/mol bis 120 000 g/mol, jeweils bezogen auf freie Säure. Ein besonders bevorzugtes Acrylsäure-Maleinsäure-Copolymer weist eine relative mittlere Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/ oder ein Vinylalkohol-Derivat oder ein Kohlenhydrat enthalten. Das erste saure Monomer beziehungsweise dessen Salz leitet sich von einer monoethylenisch ungesättigten $C_3$-$C_8$-Carbonsäure und vorzugsweise von einer $C_3$-$C_4$-Monocarbonsäure, insbesondere von (Meth)-acrylsäure ab. Das zweite saure Monomer beziehungsweise dessen Salz kann ein Derivat einer $C_4$-$C_8$-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von $C_1$-$C_4$-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem $C_1$-$C_4$-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere weisen im Allgemeinen eine relative mittlere Molekülmasse zwischen 1 000 g/mol und 200 000 g/mol, vorzugsweise zwischen 200 g/mol und 50 000 g/mol auf. Weitere bevorzugte Copolymere sind solche, die als Monomere Acrolein und Acrylsäure/Acrylsäuresalze beziehungsweise Vinylacetat aufweisen. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt.

Als wasserlösliche anorganische Buildermaterialien kommen insbesondere Polyphosphate, vorzugsweise Natriumtriphosphat, in Betracht. Als wasserunlösliche anorganische Buildermaterialien werden insbesondere kristalline oder amorphe, wasserdispergierbare Alkalialumosilikate, in Mengen nicht über 25 Gew.-%, vorzugsweise von 3 Gew.-% bis 20 Gew.-% und insbesondere in Mengen von 5 Gew.-% bis 15 Gew.-% eingesetzt. Unter diesen sind die kristallinen Natriumalumosilikate in Waschmittelqualität, insbesondere Zeolith A, Zeolith P sowie Zeolith MAP und gegebenenfalls Zeolith X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 $\mu$m auf und bestehen vorzugsweise zu wenigstens 80 Gew.-% aus Teilchen mit einer Größe unter 10 $\mu$m. Ihr Calciumbindevermögen liegt in der Regel im Bereich von 100 bis 200 mg CaO pro Gramm.

Zusätzlich oder alternativ zum genannten wasserunlöslichen Alumosilikat und Alkalicarbonat können weitere wasserlösliche anorganische Buildermaterialien enthalten sein. Zu diesen gehören neben den Polyphosphaten wie Natriumtriphosphat insbesondere die wasserlöslichen kristallinen und/oder amorphen Alkalisilikat-Builder. Derartige wasserlösliche anorganische Buildermaterialien sind in den Mitteln vorzugsweise in Mengen von 1 Gew.-% bis 20 Gew.-%, insbesondere von 5 Gew.-% bis 15 Gew.-% enthalten. Die als Buildermaterialien brauchbaren Alkalisilikate weisen vorzugsweise ein molares Verhältnis von Alkalioxid zu $SiO_2$ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis $Na_2O:SiO_2$ von 1:2 bis 1:2,8. Als kristalline Silikate, die allein oder im Gemisch mit amorphen Silikaten vorliegen können, werden vorzugsweise kristalline Schichtsilikate der allgemeinen Formel $Na_2Si_xO_{2x+1} \cdot y\ H_2O$ eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch $\delta$-Natriumdisitikate ($Na_2Si_2O_5 \cdot y\ H_2O$) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in den Mitteln eingesetzt werden. In einer weiteren bevorzugten Ausführungsform wird ein kristallines Natriumschichtsilikat mit einem Modul von 2 bis 3 eingesetzt, wie es aus Sand und Soda hergestellt werden kann. Natriumsilikate mit einem Modul im Bereich von 1,9 bis 3,5 werden in einer weiteren Ausführungsform eingesetzt. In einer bevorzugten Ausgestaltung solcher Mittel setzt man ein granulares Compound aus Alkalisilikat und Alkalicarbonat ein, wie es zum Beispiel unter dem Namen Nabion® 15 im Handel erhältlich ist.

Als Bleichmittel kommen solche auf Chlorbasis, wie insbesondere Alkalihypochlorit, Dichlorisocyanursäure, Trichlorisocyanursäure und deren Salze, insbesondere aber auch solche auf Persauerstoffbasis in Frage. Als geeignete Persauerstoffverbindungen kommen insbesondere organische Persäuren beziehungsweise persaure Salze organischer Säuren, wie Phthalimidopercapronsäure, Perbenzoesäure, Monoperoxyphthalsäure, und Diperdodecandisäure sowie deren Salze wie Magnesiummonoperoxyphthalat, Wasserstoffperoxid und unter den Einsatzbbedingungen Wasserstoffperoxid abgebende anorganische Salze, wie Perborat, Percarbonat und/oder Persilikat, und Wasserstoffperoxid-Einschlußverbindungen, wie $H_2O_2$-Harnstoffaddukte, in Betracht. Wasserstoffperoxid kann dabei auch mit Hilfe eines enzymatischen Systems, das heißt einer Oxidase und ihres Substrats, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder Wasserstoffperoxid in Form wäßriger Lösungen, die 3 Gew.-% bis 10 Gew.-% Wasserstoffperoxid enthalten, eingesetzt. Falls ein Waschmittel Persauerstoffverbindungen enthält, sind diese in Mengen von vorzugsweise bis zu 25 Gew.-%, insbesondere von 1 Gew.-% bis 20 Gew.-% und besonders bevorzugt von 7 Gew.-% bis 20 Gew.-% vorhanden.

Als bleichaktivierende, unter Perhydrolysebedingungen Peroxocarbonsäure-liefernde Verbindung können insbesondere Verbindungen, die unter Perhydrolysebedingungen gegebenenfalls substituierte Perbenzoesäure und/oder aliphatische Peroxocarbonsäuren mit 1 bis 12 C-Atomen, insbesondere 2 bis 4 C-Atomen ergeben, allein oder in Mischungen, eingesetzt werden. Geeignet Bleichaktivatoren, die O- und/oder N-Acylgruppen insbesondere der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate, insbesondere 1,5-Diacetyl-2,4-dioxohexa-hydro-1,3,5-triazin (DADHT), N-Acylimide, insbesondere N-Nonanoylsuccinimid (NOSI), acylierte Phenolsulfonate oder -carboxylate beziehungsweise die Sulfon- oder Carbonsäuren von diesen, insbesondere Nonanoyl- oder Isononanoyl- oder Lauroyloxybenzolsulfonat (NOBS beziehungsweise iso-NOBS beziehungsweise LOBS) oder Decanoyloxybenzoat (DOBA), deren formale Kohlensäureesterderivate wie 4-(2-Decanoyloxyethoxycarbonyloxy)-benzolsulfonat (DECOBS), acylierte mehrwertige Alkohole, insbesondere Triacetin, Ethylenglykoldiacetat und 2,5-Diacetoxy-2,5-dihydrofuran sowie acetyliertes Sorbitol und Mannitol und deren Mischungen (SORMAN), acylierte Zuckerderivate, insbesondere Pentaacetylglukose (PAG), Pentaacetylfruktose, Tetraacetylxylose und Octaacetyllactose, acetyliertes, gegebenenfalls N-alkyliertes Glucamin und Gluconolacton, und/oder N-acylierte Lactame, beispielsweise N-Benzoylcaprolactam.

Zusätzlich zu den Verbindungen, die unter Perhydrolysebedingungen Peroxocarbonsäuren bilden, können weitere bleichaktivierende Verbindungen, wie beispielsweise Nitrile, aus denen sich unter Perhydrolysebedingungen Perimidsäuren bilden, vorhanden sein. Dazu gehören insbesondere Aminoacetonitrilderivate mit quaterniertem Stickstoffatom gemäß der Formel

$$\begin{array}{l} \quad R^1 \\ \quad | \\ R^2\text{-}N^{(+)}\text{-}(CR^4R^5)\text{-}CN \qquad X^{(-)} \\ \quad | \\ \quad R^3 \end{array}$$

in der $R^1$ für -H, $-CH_3$, einen $C_{2-24}$-Alkyl- oder -Alkenylrest, einen substituierten $C_{1-24}$-Alkyl- oder $C_{2-24}$-Alkenylrest mit mindestens einem Substituenten aus der Gruppe -Cl, -Br, -OH, $-NH_2$, -CN und $-N^{(+)}-CH_2-CN$, einen Alkyl- oder Alkenylarylrest mit einer $C_{1-24}$-Alkylgruppe, oder für einen substituierten Alkyl- oder Alkenylarylrest mit mindestens einer, vorzugsweise zwei, gegebenenfalls substituierten $C_{1-24}$-Alkylgruppe(n) und gegebenenfalls weiteren Substituenten am aromatischen Ring steht, $R^2$ und $R^3$ unabhängig voneinander ausgewählt sind aus $-CH_2-CN$, $-CH_3$, $-CH_2-CH_3$, $-CH_2-CH_2-CH_3$, $-CH(CH_3)-CH_3$,$-CH_2-OH$, $-CH_2-CH_2-OH$, $-CH(OH)-CH_3$, $-CH_2-CH_2-CH_2-OH$,$-CH_2-CH(OH)-CH_3$, $-CH(OH)-CH_2-CH_3$, $-(CH_2CH_2-O)_nH$ mit n = 1, 2, 3, 4, 5 oder 6, $R^4$ und $R^5$ unabhängig voneinander eine voranstehend für $R^1$, $R^2$ oder $R^3$ angegebene Bedeutung haben, wobei mindestens 2 der genannten Reste, insbesondere $R^2$ und $R^3$, auch unter Einschluß des Stickstoffatoms und gegebenenfalls weiterer Heteroatome ringschließend miteinander verknüpft sein können und dann vorzugsweise einen Morpholino-Ring ausbilden, und X ein ladungsausgleichendes Anion, vorzugsweise ausgewählt aus Benzolsulfonat, Toluolsulfonat, Cumolsulfonat, den $C_{9-15}$-Alkylbenzolsulfonaten, den $C_{1-20}$-Alkylsulfaten, den $C_{8-22}$-Carbonsäuremethylestersulfonaten, Sulfat, Hydrogensulfat und deren Gemischen, ist, können eingesetzt werden. Auch sauerstoffübertragende Sulfonimine und/oder Acylhydrazone können eingesetzt werden.

Auch die Anwesenheit von bleichkatalysierenden Übergangsmetallkomplexen ist möglich. Diese werden vorzugsweise unter den Cobalt-, Eisen-, Kupfer-, Titan-, Vanadium-, Mangan- und Rutheniumkomplexen ausgewählt. Als Liganden in derartigen Übergangsmetallkomplexen kommen sowohl anorganische als auch organische Verbindungen in Frage, zu denen neben Carboxylaten insbesondere Verbindungen mit primären, sekundären und/oder tertiären Amin- und/oder Alkohol-Funktionen, wie Pyridin, Pyridazin, Pyrimidin, Pyrazin, Imidazol, Pyrazol, Triazol, 2,2'-Bispyridylamin, Tris-(2-pyridylmethyl)amin, 1,4,7-Triazacyclononan, 1,4,7-Trimethyl-1,4,7-triazacyclononan, 1,5,9-Trimethyl-1,5,9-triazacyclododecan, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimidazolylmethyl)aminomethyl)-4-methylphenol, N,N,N',N'-Tetrakis-(2-benzimidazolylmethyl)-2-hydroxy-1,3-diaminopropan, 2,6-Bis-(bis-(2-pyridylmethyl)aminomethyl)-4-methylphenol, 1,3-Bis-(bis-(2-benzimidazolylmethyl)aminomethyl)-benzol, Sorbitol, Mannitol, Erythritol, Adonitol, Inositol, Lactose, und gegebenenfalls substituierte Salene, Porphine und Porphyrine gehören. Zu den anorganischen Neutralliganden gehören insbesondere Ammoniak und Wasser. Falls nicht sämtliche Koordinationsstellen des Übergangsmetallzentralatoms durch Neutralliganden besetzt sind, enthält der Komplex weitere, vorzugsweise anionische und unter diesen insbesondere ein- oder zweizähnige Liganden. Zu diesen gehören insbesondere die Halogenide wie Fluorid, Chlorid, Bromid und Iodid, und die $(NO_2)^-$-Gruppe, das heißt ein Nitro-Ligand oder ein Nitrito-Ligand. Die $(NO_2)^-$-Gruppe kann an ein Übergangsmetall auch chelatbildend gebunden sein oder sie kann zwei Übergangsmetallatome asymmetrisch oder $\eta^1$-O-verbrücken. Außer den genannten Liganden können die Übergangsmetallkomplexe noch weitere, in der Regel einfacher aufgebaute Liganden, insbesondere ein- oder mehrwertige Anionliganden, tragen. In Frage kommen beispielsweise Nitrat, Actetat, Trifluoracetat, Formiat, Carbonat, Citrat, Oxalat, Perchlorat sowie komplexe Anionen wie Hexafluorophosphat. Die Anionliganden sollen für den Ladungsausgleich zwischen Übergangsmetall-Zentralatom und dem Ligandensystem sorgen. Auch die Anwesenheit von Oxo-Liganden, Peroxo-Liganden und Imino-Liganden ist möglich. Insbesondere derartige Liganden können auch verbrückend wirken, so daß mehrkernige Komplexe entstehen. Im Falle verbrückter, zweikerniger Komplexe müssen nicht beide Metallatome im Komplex gleich sein. Auch der Einsatz zweikerniger Komplexe, in denen die beiden Übergangsmetallzentralatome unterschiedliche Oxidationszahlen aufweisen, ist möglich. Falls Anionliganden fehlen oder die Anwesenheit von Anionliganden nicht zum Ladungsausgleich im Komplex führt, sind in den gemäß der Erfindung zu verwendenden Übergangsmetallkomplex-Verbindungen anionische Gegenionen anwesend, die den kationischen Übergangsmetall-Komplex neutralisieren. Zu diesen anionischen Gegenionen gehören insbesondere Nitrat, Hydroxid, Hexafluorophosphat, Sulfat, Chlorat, Perchlorat, die Halogenide wie Chlorid oder die Anionen von Carbonsäuren wie Formiat, Acetat, Oxalat, Benzoat oder Citrat. Beispiele für einsetzbare Übergangsmetallkomplex-Verbindungen sind $Mn(IV)_2(\mu\text{-}O)_3$(1,4,7-trimethyl-1,4,7-triazacyclononan)-di-hexafluorophosphat, [N,N'-Bis[(2-hydroxy-5-vinylphenyl)-methylen]-1,2-diaminocyclohexan]-mangan-(III)-chlorid, [N,N'-Bis[(2-hydroxy-5-nitrophenyl)-methylen]-1,2-diaminocyclohexan]-mangan-(III)-acetat, [N,N'-Bis[(2-hydroxyphenyl)-methylen]-1,2-phenylendiamin]-mangan-(III)-acetat, [N,N'-Bis[(2-hydroxyphenyl)-methylen]-1,2-diaminocyclohexan]-mangan-(III)-chlorid, [N,N'-Bis[(2-hydroxyphenyl)-methylen]-1,2-diaminoethan]-mangan-(III)-chlorid, [N,N'-Bis[(2-hydroxy-5-sulfonatophenyl)-methylen]-1,2-diaminoethan]-mangan-(III)-chlorid, Mangan-oxalatokomplexe, Nitropentammin-cobalt(III)-chlorid, Nitritopentammin-cobalt(III)-chlorid, Hexammincobalt(III)-chlorid, Chloropentammin-cobalt(III)-chlorid sowie der Peroxo-Komplex $[(NH_3)_5Co\text{-}O\text{-}O\text{-}Co(NH_3)_5]Cl_4$.

Als in den Mitteln zusätzlich zu den erfindungsgemäßen Laccasen verwendbare Enzyme kommen solche aus der Klasse der Amylasen, Proteasen, Lipasen, Cutinasen, Pullulanasen, Hemicellulasen, Cellulasen, Oxidasen und Peroxidasen sowie deren Gemische in Frage. Auch der Einsatz einer oder mehrer weiterer Laccasen bzw. Multi-Kupfer-Oxidasen zusätzlich zu den erfindungsgemäßen Laccasen ist erfindungsgemäß möglich. Besonders geeignet sind aus Pilzen oder Bakterien, wie Bacillus subtilis, Bacillus licheniformis, Bacillus lentus, Streptomyces griseus, Humicola lanuginosa, Humicola insolens, Pseudomonas pseudoalcaligenes, Pseudomonas cepacia oder Coprinus cinereus gewonnene enzymatische Wirkstoffe. Die Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- oder Reinigungsmitteln vorzugsweise in Mengen bis zu 5 Gew.-%, insbesondere von 0,2 Gew.-% bis 4 Gew.-%, enthalten. Falls das erfindungsgemäße Mittel Protease enthält, weist es vorzugsweise eine proteolytische Aktivität im Bereich von etwa 100 PE/g bis etwa 10 000 PE/g, insbesondere 300 PE/g bis 8000 PE/g auf. Falls mehrere Enzyme in dem erfindungsgemäßen Mittel eingesetzt werden sollen, kann dies durch Einarbeitung der zwei oder mehreren separaten beziehungsweise in bekannter Weise separat konfektionierten Enzyme oder durch zwei oder mehrere gemeinsam in einem Granulat konfektionierte Enzyme durchgeführt werden.

Zu den in den Waschmitteln, insbesondere wenn sie in flüssiger oder pastöser Form vorliegen, neben Wasser verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Mitteln in Mengen von vorzugsweise nicht über 20 Gew.-%, insbesondere von 1,2 Gew.-% bis 17 Gew.-%, enthalten.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt.

Waschmittel können als optische Aufheller beispielsweise Derivate der Diaminostilbendisulfonsäure beziehungsweise deren Alkalimetallsalze enthalten, obgleich sie für den Einsatz als Colorwaschmittel vorzugsweise frei von optischen Aufhellern sind. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Verfahren kann es von Vorteil sein, den Mitteln übliche Schauminhibitoren zuzusetzen. Als Schauminhibitoren eignen sich beispielsweise Seifen natürlicher oder synthetischer Herkunft, die einen hohen Anteil an $C_{18}$-$C_{24}$-Fettsäuren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

Die Herstellung fester Mittel bietet keine Schwierigkeiten und kann auf bekannte Weise, zum Beispiel durch Sprühtrocknen oder Granulation, erfolgen, wobei Enzyme und eventuelle weitere thermisch empfindliche Inhaltsstoffe

wie zum Beispiel Bleichmittel gegebenenfalls später separat zugesetzt werden. Zur Herstellung von Mitteln mit erhöhtem Schüttgewicht, insbesondere im Bereich von 650 g/l bis 950 g/l, ist ein einen Extrusionschritt aufweisendes Verfahren bevorzugt.

Zur Herstellung von Mitteln in Tablettenform, die einphasig oder mehrphasig, einfarbig oder mehrfarbig und insbesondere aus einer Schicht oder aus mehreren, insbesondere aus zwei Schichten bestehen können, geht man vorzugsweise derart vor, dass man alle Bestandteile - gegebenenfalls je einer Schicht - in einem Mischer miteinander vermischt und das Gemisch mittels herkömmlicher Tablettenpressen, beispielsweise Exzenterpressen oder Rundläuferpressen, mit Preßkräften im Bereich von etwa 50 bis 100 kN, vorzugsweise bei 60 bis 70 kN verpreßt. Insbesondere bei mehrschichtigen Tabletten kann es von Vorteil sein, wenn mindestens eine Schicht vorverpreßt wird. Dies wird vorzugsweise bei Preßkräften zwischen 5 und 20 kN, insbesondere bei 10 bis 15 kN durchgeführt. Man erhält so problemlos bruchfeste und dennoch unter Anwendungsbedingungen ausreichend schnell lösliche Tabletten mit Bruch- und Biegefestigkeiten von normalerweise 100 bis 200 N, bevorzugt jedoch über 150 N. Vorzugsweise weist eine derart hergestellte Tablette ein Gewicht von 10 g bis 50 g, insbesondere von 15 g bis 40 g auf. Die Raumform der Tabletten ist beliebig und kann rund, oval oder eckig sein, wobei auch Zwischenformen möglich sind. Ecken und Kanten sind vorteilhafterweise abgerundet. Runde Tabletten weisen vorzugsweise einen Durchmesser von 30 mm bis 40 mm auf. Insbesondere die Größe von eckig oder quaderförmig gestalteten Tabletten, welche überwiegend über die Dosiervorrichtung der Waschmaschine eingebracht werden, ist abhängig von der Geometrie und dem Volumen dieser Dosiervorrichtung. Beispielhaft bevorzugte Ausführungsformen weisen eine Grundfläche von (20 bis 30 mm) x (34 bis 40 mm), insbesondere von 26x36 mm oder von 24x38 mm auf.

Flüssige beziehungsweise pastöse Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

Erfindungsgemäße feste und/oder flüssige Waschmittel können z. B. auch in Portions-Säckchen oder (vorzugsweise selbstauflösenden) Portionsbeuteln (pouches) abgepackt sein, insbesondere auch in Mehrkammerpouches. Unter den Begriff der Flüssigkeit fallen im Sinne der Erfindung auch jegliche Festkörperdispersionen in Flüssigkeiten. Erfindungsgemäße flüssige Mittel können auch mehrphasig sein, die Phasen können z. B. vertikal, also übereinander oder horizontal, also nebeneinander angeordnet sein.

Beispiele:

Die folgenden Beispiele erläutern die Erfindung, ohne sie jedoch darauf einzuschränken:

Beispiel 1:

Aktivitätsbestimmung der Laccasen mit den Aminosäuresequenzen SEQ ID NO. 1, SEQ ID NO. 3, SEQ ID NO. 5 und SEQ ID NO. 7:

Malvidin-Assay: Die Bestimmung der Enzymaktivität der Laccase erfolgte fotometrisch. Hierfür wurde als Laccasesubstrat das Anthocyanin Primulin (Malvidin-3-galactoside chloride, CAS Number: 30113-37-2) verwendet.

Die Absorption wurde in einem Synergy 2™ microplate reader (BioTek Instruments GmbH, Bad Friedrichshall, Germany) gemessen.

Die Substratlösung bestand aus Puffer (0.1 M NaAc; 0.1 M Kaliumphosphat, pH 8) und 31 mmol Primulin (Sigma). 50 µL der Laccaseprobe wurden zu 100 µL Substratlösung in eine Mikrotiterplatte gegeben und die Absorptionsabnahme über 30 Minuten bei 30 °C als -mAbs/min gemessen. Vergleichsproben wurden mit Wasser anstelle der Laccaseprobe hergestellt.

Umrechnung in Units:

Die Berechnung der Enzymaktivität erfolgte an Hand der Formel:

Aktivität [ Malvidin mU/mL] = (Gesamtvolumen im Assay in µL* Steigung pro min) / (Probevolumen im Assay in mL * 16000L/mol/cm*Schichtdicke in cm)

Die Schichtdicke mit 150 µL Füllmenge in den Mikrotiterplatten betrug 0,425 cm. Die negative Steigung (-Abs/min) wurde positiv gesetzt.

Mit dieser Methode werden die Aktivitäten der aus den Wildtypüberständen aufgereinigten Laccasen bestimmt, welche für Beispiel 2 benutzt werden:

Die Enzymprobe des Pleurotus pulmonarius aufgereinigten Wildtypüberstandes zeigt eine Aktivität von -21,4 mAbs/min

und somit 9 Malvidin mU/mL.

Die Enzymprobe des Pleurotus ostreatus var. Florida aufgereinigten Wildtypüberstandes zeigt eine Aktivität von -20,4 mAbs/min und somit 9 Malvidin mU/mL.

Die Enzymprobe des Lentinula edodes aufgereinigten Wildtypüberstandes zeigt eine Aktivität von - 4,5 mAbs/min und somit 2 Malvidin mU/mL.

Die Enzymprobe des Trametes species (Tsp, DSMZ 11309) aufgereinigten Wildtypüberstandes zeigt eine Aktivität von -29,5 mAbs/min und somit 13 Malvidin mU/mL.

ABTS-Assay:

Für den ABTS Assay werden in einer MTP 245 μL 50 mM Na-Acetat Puffer pH 4,5 vorgelegt, plus 30 μL 5 mM ABTS Lösung, plus 10 μL 2 mM H2O2 plus 15 μL Probe in entsprechender Verdünnung. Es wird eine kinetische Messung bei 420 nm, 30°C für 10 min gemacht. Über den molaren Extinktionskoeffizienten für ABTS lässt sich die Aktivität in μmol freigesetzte ABTS-Radikale pro Minute und ml berechnen

Probenvolumen: 0,015 mL, Testvolumen: 0,3 mL, eABTS = 0,0368cm2*μmol-1, bei 405-420nm

Aktivität [μmol*min-1*ml-1]=(((DE/minProbe-DE/minBlank)*p*r2)/(eABTS*Testvolumen))*(Testvolumen/Probenvolumen)*Verdünnungsfaktor

Mit dieser Methode werden in Trichoderma heterolog exprimierte Laccasen bestimmt, welche für Beispiel 4 benutzt werden:

| | |
|---|---|
| Laccase aus Led | 2359 ABTS U/mL |
| Laccase aus Ppu | 6152 ABTS U/mL |
| Laccase aus Tsp | 3142 ABTS U/mL |

Beispiel 2:

Erste Mini-Waschtests erfolgten jeweils mit einem der folgenden aufgereinigten Wildtyp-Überständen:

a) Pleurotus pulmonarius, in dem die Laccase mit der Aminosäuresequenz SEQ ID NO. 1 vorliegt.
b) Pleurotus ostreatus var. florida, in dem die Laccase mit der Aminosäuresequenz SEQ ID NO. 3 vorliegt;
c) Lentinula edodes, in dem die Laccase mit der Aminosäuresequenz SEQ ID NO. 5 vorliegt;
d) Trametes species (Tsp, DSMZ 11309), in dem die Laccase mit der Aminosäuresequenz SEQ ID NO. 7 vorliegt

Die Testbedingungen waren wie folgt: 40°C, 16°dH Wasser, 1 h, Laccasekonzentration: 1 Malvidin mU/mL

Für diesen Test wurden standardisiert verschmutzte Textilien eingesetzt. Es wurden folgende Anschmutzungen verwendet:

- Johannisbeere CS12 [CFT (Center For Testmaterials) B.V. Vlaardingen, Niederlande]
- Blaubeere CS15 [CFT (Center For Testmaterials) B.V. Vlaardingen, Niederlande]
- Tee EMPA 167 [EMPA: Eidgenössische Material- und Prüfanstalt (EMPA) Testmaterialien AG [Federal materials and testing agency, Testmaterials], St. Gallen, Switzerland]

Die ausgestanzten Gewebe (Durchmesser = 10 mm) wurden in Mikrotiterplatten vorlegt, die Waschlauge wurde auf 40°C vortemperiert, Die Endkonzentration der Waschmatrix betrug 4,06 g/L in 16°dH Wasser.

Dann wurden Lauge und Enzym auf die Anschmutzung gegeben, gefolgt von einer Inkubation für 1 h bei 40°C und 600 rpm.

Abschließend wurde die Anschmutzung mehrmals mit klarem Wasser gespült, trocknen gelassen und die Helligkeit wurde mit einem Minolta Farbmeßgerät Cm700d (CIE L*a*b*, D65/100/SCI) bestimmt.

Die Reinigungsleistung zeigte sich an der Helligkeit des gewaschenen Gewebes. Je heller das Gewebe wurde, desto besser war die Reinigungsleistung. Gemessen wurde der L-Wert = Helligkeit, je höher desto heller.

Es wurde mit einem Flüssigwaschmittel gewaschen, das keine Enzyme, optische Aufheller und Farbstoffe enthielt. Die Rezeptur ist in Beispiel 3 angegeben.

a) Waschtest mit Laccase aus Trametes species (Tsp, DSMZ 11309)

Probe 1: nur Waschmittel als Benchmark
Probe 2: Waschmittel plus Laccase aus Trametes species (Tsp, DSMZ 11309), 1 mU/mL
Probe 3: Waschmittel plus Laccase aus Pleurotus ostreatus (Sigma), 1 mU/mL

Ergebnis (Probe 1 vs. Probe 2):

| Anschmutzung | Probe 1 | Probe 2 |
|---|---|---|
| Johannisbeere | 74,8 | 79,7 |
| Blaubeere | 73,3 | 74,0 |
| Tee | 73,8 | 75,1 |

Man erkennt deutlich, dass die Laccase auf beiden Anthocyan-haltigen Anschmutzungen (Johannisbeere und Blaubeere) eine gute Leistung zeigt. Der Tee Fleck wurde nicht verdunkelt, wie dies von anderen Laccasen bekannt ist, sondern vorteilhafterweise deutlich aufgehellt.
Von einer signifikanten Leistungsverbesserung kann bereits ab 1 Einheit gesprochen werden; hier wurden sogar 4,9 Einheiten Verbesserung erzielt.
Als Negativkontrolle wurde eine käufliche Laccase aus Pleurotus ostreatus auf gleichem Aktivitätslevel eingesetzt wie die Laccase in Probe 2.

Ergebnis (Probe 1 vs. Probe 3):

| Anschmutzung | Probe 1 | Probe 3 |
|---|---|---|
| Johannisbeere | 76,8 | 77,7 |
| Blaubeere | 76,4 | 75,7 |
| Tee | 75,1 | 74,8 |

Die Waschleistung der Probe 3 ist gegenüber Probe 1 erkennbar nicht verbessert.

b) Waschtest mit Laccase aus Pleurotus ostreatus var. Florida (Pos)

Probe 1: nur Waschmittel als Benchmark
Probe 2: Waschmittel plus Laccase aus Pos, 1 mU/mL
Probe 3: Waschmittel plus Laccase aus Pleurotus ostreatus (Sigma), 1 mU/mL

Ergebnis (Probe 1 vs. Probe 2):

| Anschmutzung | Probe 1 | Probe 2 |
|---|---|---|
| Johannisbeere | 74,8 | 78,1 |
| Blaubeere | 73,3 | 75,7 |
| Tee | 73,8 | 74,1 |

Man erkennt deutlich, dass die Laccase auf beiden Anthocyan-haltigen Anschmutzungen (Johannisbeere und Blaubeere) eine gute Leistung zeigt, und keine Verdunklung auf Tee, wie sie viele andere Laccasen bewirken. Von einer signifikanten Leistungsverbesserung kann bereits ab 1 Einheit gesprochen werden; hier wurden sogar 3,3 bzw. 2,4 Einheiten Verbesserung erzielt.
Für die Negativkontrolle siehe a).

c) Waschtest mit Laccase aus Lentinula edodes

Probe 1: nur Waschmittel als Benchmark
Probe 2: Waschmittel plus Laccase aus Lentinula edodes (Led), 1 mU/mL
Probe 3: Waschmittel plus Laccase aus Pleurotus ostreatus (Sigma), 1 mU/mL

Ergebnis (Probe 1 vs. Probe 2):

| Anschmutzung | Probe 1 | Probe 2 |
|---|---|---|
| Johannisbeere | 74,8 | 79,5 |
| Blaubeere | 73,3 | 76,9 |
| Tee | 73,8 | 77,0 |

Man erkennt deutlich, dass die Laccase auf beiden Anthocyan-haltigen Anschmutzungen (Johannisbeere und Blaubeere) eine gute Leistung zeigt. Der Teefleck wurde nicht nur nicht verdunkelt, wie dies von anderen Laccasen bekannt ist, er wurde sogar vorteilhafterweise deutlich aufgehellt. Von einer signifikanten Leistungsverbesserung kann bereits ab 1 Einheit gesprochen werden; hier wurden sogar 4,7 bzw. 3,4 Einheiten Verbesserung erzielt.
Für die Negativkontrolle siehe a).

d) Waschtest mit Laccase aus Pleurotus pulmonarius

Probe 1: nur Waschmittel als Benchmark
Probe 2: Waschmittel plus Laccase aus Pleurotus pulmonarius (Ppu), 1 mU/mL
Probe 3: Waschmittel plus Laccase aus Pleurotus ostreatus (Sigma), 1 mU/mL

Ergebnis (Probe 1 vs. Probe 2):

| Anschmutzung | Probe 1 | Probe 2 |
|---|---|---|
| Johannisbeere | 74,8 | 77,4 |
| Blaubeere | 73,3 | 76,6 |
| Tee | 73,8 | 74,3 |

Man erkennt deutlich, dass die Laccase auf beiden Anthocyan-haltigen Anschmutzungen (Johannisbeere und Blaubeere) eine gute Leistung zeigt. Der Tee Fleck wurde nicht nur nicht verdunkelt, wie dies von anderen Laccasen bekannt ist, er wurde sogar vorteilhafterweise leicht aufgehellt. Von einer signifikanten Leistungsverbesserung kann bereits ab 1 Einheit gesprochen werden; hier wurden sogar 2,6 bzw. 3,3 Einheiten Verbesserung erzielt.
Für die Negativkontrolle siehe a).

Beispiel 3:

Waschmittelformulierung:

Diese Rezeptur wurde in Beispiel 2 benutzt. Die Dosierung betrug 69g/17L:

| Chemischer name | % Aktivsubstanz (Rohmaterial) | % Aktivsubstanz in Formulierung |
|---|---|---|
| Wasser demin. | 100 | Rest |
| Alkylbenzolsulfonsäure | 96 | 4,40 |

(fortgesetzt)

| Chemischer name | % Aktivsubstanz (Rohmaterial) | % Aktivsubstanz in Formulierung |
|---|---|---|
| Anionisches Tensid | 70 | 5,60 |
| C12-C18 Fettsäure Na-Salz | 100 | 2,40 |
| Nonionisches Tensid | 100 | 4,40 |
| Phosphonat | 32 | 0,20 |
| Zitronensäure | 100 | 1,43 |
| NaOH | 50 | 0,95 |
| Antischaum | 10 | 0,01 |
| Glycerin | 99,5 | 2,00 |
| Konservierungsmittel | 100 | 0,08 |
| Ethanol | 93 | 1,00 |
| Ohne opt. Aufheller, Parfüm, Farbstoff und Enzyme. | | |

Beispiel 4:

Waschversuch im Launder-O-meter:

Die Testbedingungen waren wie folgt:

- 40°C, 16°dH Wasser, 1 h, 200 mL Waschflotte, 10 Stahlkugeln, 16,8 g Anschmutzungen und Füllgewebe
- Laccasekonzentration: 25 ABTS U/mL

Für diesen Test wurden hauptsächlich natürliche Anschmutzungen benutzt, hergestellt aus Säften und Marmeladen. Es wurden folgende Anschmutzungen verwendet:

- Wildheidelbeer Konfitüre, Darbo
- Baseler Schwarzkirsche Marmelade, Mövenpick
- Heidelbeer-Saft, Rabenhorst
- Cranberry-Saft, Rabenhorst
- Preiselbeer-Saft, Rabenhorst
- Holunderbeer-Saft, Rabenhorst
- Blaubeere CS15, [CFT (Center For Testmaterials) B.V. Vlaardingen, Niederlande]

Die Anschmutzungen werden auf Baumwollgewebe aufgetragen (ca. 5 cm Durchmesser), eingebürstet und für mindestens 1 Woche gelagert.
Nachdem das Launder-o-meter 1 h bei 40°C gelaufen ist werden die Anschmutzungen 4 x mit Wasser gespült, anschließend geschleudert und Luft-getrocknet. Die Messung der Helligkeit erfolgt nach dem Mangeln mit einem Minolta CR400-1 Farbmessgerät. Es wird der Farbwert-Y bestimmt, im Vergleich zum Blank (Waschmittel ohne Enzyme).
Für den Waschtest wurden die folgenden 3 Laccasen benutzt, welche heterolog in Trichoderma exprimiert wurden:

a) Laccase aus Pleurotus pulmonarius (Ppu), mit SEQ ID NO. 1
b) Laccase aus Lentinula edodes (Led), mit SEQ ID NO. 5
c) Laccase aus Trametes species (Tsp) (Tsp, DSMZ 11309), mit SEQ ID NO. 7.

Ergebnis:

Die Werte stellen das delta-Y zum Waschmittel Blank ohne Enzyme dar. Von einer signifikanten Verbesserung spricht man ab 1 Einheit Änderung.

| | Laccase aus Led | Laccase aus Ppu | Laccase aus Tsp |
|---|---|---|---|
| Wildheidelbeer Konfitüre | 1,5 | 2,2 | 2,4 |
| Baseler Schwarzkirsch Marmelade | 1,8 | 1,5 | 2,0 |
| Heidelbeer-Saft | 0,4 | 1,7 | 0,4 |
| Cranberry-Saft | 0,6 | 1,1 | 1,0 |
| Preiselbeer-Saft | 0,8 | 1,5 | 0,4 |
| Holunderbeer-Saft | 1,0 | 1,1 | 1,0 |
| CS-15 Blaubeere | 1,3 | 1,8 | 3,0 |

Man erkennt auf einigen Anthocyan haltigen Flecken eine signifikante Reinigungsleistung. Die Laccase aus Pleurotus pulmonarius zeigt das breiteste Leistungsspektrum.

**Patentansprüche**

1. Laccase, umfassend eine Aminosäuresequenz, die zu der in SEQ ID NO. 1 angegebenen Aminosäuresequenz über deren Gesamtlänge zu mindestens 97,5% und zunehmend bevorzugt zu mindestens 98%, 98,5% und 99% identisch ist.

2. Laccase, **dadurch gekennzeichnet, dass** sie durch eine Nukleinsäuresequenz kodiert wird, die zu der in SEQ ID NO. 2 angegebenen Nukleinsäuresequenz über deren Gesamtlänge zu mindestens 94,5% und zunehmend bevorzugt zu mindestens 95%, 95,5%, 96%, 96,5%, 97%, 97,5%, 98%, 98,5% und 99% identisch ist.

3. Waschmittel, enthaltend mindestens
eine Laccase, die eine Aminosäuresequenz umfasst, die zu der in SEQ ID NO. 1 angegebenen Aminosäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, oder
eine Laccase, die durch eine Nukleinsäuresequenz kodiert wird, die zu der in SEQ ID NO. 2 angegebenen Nukleinsäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist.

4. Waschmittel nach Anspruch 3, wobei die mindestens eine Laccase eine Laccase nach Anspruch 1 oder 2 ist.

5. Waschmittel nach Anspruch 3 oder 4, **dadurch gekennzeichnet, dass** die Konzentration der mindestens einen Laccase von 0,001 bis 0,15 Gew.-%, vorzugsweise von 0,005 bis 0,06 Gew.-%, bezogen auf aktives Protein beträgt.

6. Waschmittel nach einem der Ansprüche 3 bis 5, **dadurch gekennzeichnet, dass** es vorzugsweise im Temperaturbereich von 5°C bis 95°C, bevorzugt 20°C bis 60°C und besonders bevorzugt 30°C bis 40°C einsetzbar ist.

7. Waschmittel nach einem der Ansprüche 3 bis 6, **dadurch gekennzeichnet, dass** es zusätzliche Mediatoren enthält, die ausgewählt sind unter 2,2,6,6-Tetramethyl-1-Piperidinyloxy, 1-Hydroxybenzotriazol, 2,2'-Azinobis-3-Ethylbenzthiazol-6-Sulphonat, N-Hydroxy-Acetanilid, 2,5-Xylidin, Ethanol, Kupfer, 4-Methylcatechol, N-Hydroxyphthalimid, Gallussäure, Gerbsäure, Quercetin, Syringasäure, Guaiacol, Dimethoxybenzyl alcohol, Phenol, Violursäure, Phenolrot, Bromphenolblau, Cellulose, p-Kumarinsäure, Rooibos, o-Kresol, Dichloroindophenol, Hydroxybenzotriazol, Cycloheximid oder Vanillin.

8. Verwendung
einer Laccase, die eine Aminosäuresequenz, die zu der in SEQ ID NO. 1 angegebenen Aminosäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, oder
einer Laccase, die durch eine Nukleinsäuresequenz kodiert wird, die zu der in SEQ ID NO. 2 angegebenen Nukleinsäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist,
zur Verbesserung der Waschleistung eines Waschmittels, insbesondere bei der Entfernung farbiger Flecken und Verschmutzungen, besonders bevorzugt bei der Entfernung Anthocyan-haltiger Anschmutzungen,
wobei die Laccase vorzugsweise eine Laccase nach Anspruch 1 oder 2 ist.

9. Verfahren zum Waschen von Textilien in tensidhaltigen wässrigen Lösungen, welches **dadurch gekennzeichnet ist, dass** man eine tensidhaltige wässrige Lösung einsetzt,
die mindestens eine Laccase enthält, die eine Aminosäuresequenz, die zu der in SEQ ID NO. 1 angegebenen Aminosäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, oder
die mindestens eine Laccase enthält, die durch eine Nukleinsäuresequenz kodiert wird, die zu der in SEQ ID NO. 2 angegebenen Nukleinsäuresequenz über deren Gesamtlänge zu mindestens 70% und zunehmend bevorzugt zu mindestens 75%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist,

wobei die Laccase vorzugsweise eine Laccase nach Anspruch 1 oder 2 ist.

## Claims

1. A laccase comprising an amino acid sequence which over the entire length thereof is at least 97.5% and increasingly preferably at least 98%, 98.5% and 99% identical to the amino acid sequence specified in SEQ ID NO. 1.

2. The laccase, **characterized in that** it is encoded by a nucleic acid sequence which over the entire length thereof is at least 94.5% and increasingly preferably at least 95%, 95.5%, 96%, 96.5%, 97%, 97.5%, 98%, 98.5% and 99% identical to the nucleic acid sequence specified in SEQ ID NO. 2.

3. A washing agent containing at least one laccase which comprises an amino acid sequence which over the entire length thereof is at least 70% and increasingly preferably at least 75%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the amino acid sequence specified in SEQ ID NO. 1, or a laccase which is encoded by a nucleic acid sequence which over the entire length thereof is at least 70% and increasingly preferably at least 75%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the nucleic acid sequence specified in SEQ ID NO. 2.

4. The washing agent according to claim 3, wherein the at least one laccase is a laccase according to claim 1 or 2.

5. The washing agent according to claim 3 or 4, **characterized in that** the concentration of the at least one laccase is from 0.001 to 0.15 wt.%, preferably from 0.005 to 0.06 wt.%, based on active protein.

6. The washing agent according to one of claims 3 to 5, **characterized in that** it can preferably be used in the temperature range from 5 °C to 95 °C, more preferably from 20 °C to 60 °C and particularly preferably from 30 °C to 40 °C.

7. The washing agent according to one of claims 3 to 6, **characterized in that** it contains additional mediators, which are selected from 2,2,6,6-tetramethyl-1-piperidinyloxy, 1-hydroxybenzotriazol, 2,2'-azinobis-3-ethylbenzthiazol-6-sulfonate, N-hydroxy-acetanilide, 2,5-xylidine, ethanol, copper, 4-methylcatechol, N-hydroxyphthalimide, gallic acid, tannic acid, quercetin, syringic acid, guaiacol, dimethoxybenzyl alcohol, phenol, violuric acid, phenol red, bromophenol blue, cellulose, p-coumaric acid, rooibos, o-cresol, dichloroindophenol, hydroxybenzotriazole, cycloheximide or vanillin.

8. The use of a laccase which comprises an amino acid sequence which over the entire length thereof is at least 70% and increasingly preferably at least 75%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the amino acid sequence specified in SEQ ID NO. 1, or a laccase which is encoded by a nucleic acid sequence which over the entire length thereof is at least 70% and increasingly preferably at least 75%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the nucleic acid sequence specified in SEQ ID NO. 2, in order to improve the washing performance of a washing agent, particularly in the removal of colored marks and stains, particularly preferably in the removal of stains which contain anthocyan, wherein the laccase is preferably a laccase according to claim 1 or 2.

9. A method for washing textiles in surfactant-containing aqueous solutions, which method is **characterized in that** a surfactant-containing aqueous solution is used which contains at least one laccase which comprises an amino acid sequence which over the entire length thereof is at least 70% and increasingly preferably at least 75%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the amino acid sequence specified in SEQ ID NO. 1, or which contains at least one laccase which is encoded by a nucleic acid sequence which over the entire length thereof is at least 70% and increasingly preferably at least 75%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5% and 99% identical to the nucleic acid sequence specified in SEQ ID NO. 2, wherein the laccase is preferably a laccase according to claim 1 or 2.

## Revendications

1. Laccase comprenant une séquence d'acides aminés identique sur toute sa longueur, à au moins 97,5 % et plus préférablement à au moins 98 %, 98,5 % et 99 %, à la séquence d'acides aminés indiquée dans SEQ ID NO.1.

2. Laccase **caractérisée en ce qu'**elle est codée par une séquence d'acides nucléiques identique sur toute sa longueur, à au moins 94,5 % et plus préférablement à au moins 95 %, 95,5 %, 96 %, 96,5 %, 97 %, 97,5 %, 98 %, 98,5 % et 99 %, à la séquence d'acides nucléiques indiquée dans SEQ ID NO.2.

3. Détergent contenant au moins une laccase comprenant une séquence d'acides aminés identique sur toute sa longueur, à au moins 70 % et plus préférablement à au moins 75 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, à la séquence d'acides aminés indiquée dans SEQ ID NO.1, ou une laccase codée par une séquence d'acides nucléiques identique sur toute sa longueur, à au moins 70 % et plus préférablement à au moins 75 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, à la séquence d'acides nucléiques indiquée dans SEQ ID NO.2.

4. Détergent selon la revendication 3, dans lequel l'au moins une laccase est une laccase selon la revendication 1 ou 2.

5. Détergent selon la revendication 3 ou 4, **caractérisé en ce que** la concentration de l'au moins une laccase est de 0,001 à 0,15 % en poids, de préférence de 0,005 à 0,06 % en poids, par rapport à la protéine active.

6. Détergent selon l'une des revendications 3 à 5, **caractérisé en ce qu'**il peut être utilisé dans une plage de température comprise entre 5 °C et 95 °C, préférablement entre 20 °C et 60 °C et plus préférablement entre 30 °C et 40 °C.

7. Détergent selon l'une des revendications 3 à 6, **caractérisé en ce qu'**il contient des médiateurs supplémentaires choisis parmi le 2,2,6,6-tétraméthyl-1-pipéridinyloxy, le 1-hydroxybenzotriazole, le 2,2'-azinobis-3-éthylbenzthiazole-6-sulfonate, le N-hydroxyacétanilide, la 2,5-xylidine, l'éthanol, le cuivre, le 4-méthylcatéchol, le N-hydroxyphthalimide, l'acide gallique, l'acide tannique, la quercétine, l'acide syringa, le guaiacol, l'alcool diméthoxybenzylique, le phénol, l'acide violurique, le rouge phénol, le bleu de bromophénol, la cellulose, l'acide p-coumarique, le rooibos, l'o-crésol, le dichloroindophénol, l'hydroxybenzotriazole, le cycloheximide ou la vanilline.

8. Utilisation d'une laccase comprenant une séquence d'acides aminés identique sur toute sa longueur, à au moins 70 % et plus préférablement à au moins 75 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, à la séquence d'acides aminés indiquée dans SEQ ID NO.1, ou d'une laccase codée par une séquence d'acides nucléiques identique sur toute sa longueur, à au moins 70 % et plus préférablement à au moins 75 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, à la séquence d'acides nucléiques indiquée dans SEQ ID NO.2, destinée à améliorer le pouvoir nettoyant d'un détergent, en particulier à éliminer les taches colorées et les salissures, plus préférablement à éliminer des souillures contenant des anthocyanines, la laccase étant de préférence une laccase selon la revendication 1 ou 2.

9. Procédé de lavage de textiles dans des solutions aqueuses contenant des tensioactifs, lequel procédé est **caractérisé en ce que** l'on utilise une solution aqueuse contenant des tensioactifs, laquelle solution aqueuse contient au moins une laccase contenant une séquence d'acides aminés identique sur toute sa longueur, à au moins 70 % et plus préférablement à au moins 75 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, à la séquence d'acides aminés indiquée dans SEQ ID NO.1, ou ladite solution aqueuse contient au moins une laccase codée par une séquence d'acides nucléiques identique sur toute sa longueur, à au moins 70 % et plus préférablement à au moins 75 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 %, à la séquence d'acides nucléiques indiquée dans SEQ ID NO.2, la laccase étant de préférence une laccase selon la revendication 1 ou 2.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9711217 A
- WO 9949020 A
- DE 102015210367
- DE 102015210369
- DE 102015210368
- DE 102015210370


### In der Beschreibung aufgeführte Nicht-Patentliteratur


- **PALMIERI, G. et al.** Atypical laccaseisoenzymes from coppersupplemented Pleurotus ostreatus cultures. *EnzymeMicrob.Technol.,* 2003, vol. 33 (2-3), 220-230
- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410
- **ALTSCHUL, STEPHAN F. ; THOMAS L. MADDEN ; ALEJANDRO A. SCHAFFER ; JINGHUI ZHANG ; HHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- **CHENNA et al.** Multiple sequence alignment with the Clustal series of programs. *Nucleic Acid Research,* 2003, vol. 31, 3497-3500
- **NOTREDAME et al.** T-Coffee: A novel method for multiple sequence alignments. *J. Mol. Biol.,* 2000, vol. 302, 205-217
- **SAMBROOK, J. ; FRITSCH, E.F. ; MANIATIS, T.** Molecular cloning: a laboratory manual. Cold Spring Laboratory Press, 2001